(19) Europäisches Patentamt

European Patent Office

Office européen des brevets

(11) **EP 4 560 312 A2**

(12) **EUROPEAN PATENT APPLICATION**

(43) Date of publication:
**28.05.2025 Bulletin 2025/22**

(21) Application number: **25158334.0**

(22) Date of filing: **01.07.2020**

(51) International Patent Classification (IPC):
***G01N 29/28*** *(2006.01)*

(52) Cooperative Patent Classification (CPC):
**G01N 33/24; G01N 21/274; G01N 21/3563;
G01N 21/84; G01N 23/207; G01N 29/07;
G01N 29/28;** G01N 15/0893; G01N 2021/3595;
G01N 2021/8472; G01N 2201/1296;
G01N 2291/0232; G01N 2291/02827

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(62) Document number(s) of the earlier application(s) in
accordance with Art. 76 EPC:
**20943498.4 / 4 176 259**

(71) Applicant: **Chevron U.S.A. Inc.
San Ramon, California 94583-0806 (US)**

(72) Inventors:
• **MONTGOMERY, Paul
Pershore, WR10 3HQ (GB)**
• **JONES, Colin
Aberdeen, AB15 9AQ (GB)**

• **RATCLIFFE, Kenneth Thomas
Powys, SY21 9BW (GB)**
• **MATHIA, Eliza
Birmingham, B15 2EX (GB)**

(74) Representative: **Haseltine Lake Kempner LLP
One Portwall Square
Portwall Lane
Bristol BS1 6BH (GB)**

Remarks:
This application was filed on 17.02.2025 as a
divisional application to the application mentioned
under INID code 62.

(54) **PROPERTIES OF ROCKS**

(57) A method comprises determining a mechanical property of a rock sample taking into account (a) a respective amount of each of two or more constituent phases in the rock sample and (b) a corresponding mechanical property parameter associated with each of the two or more constituent phases.

EP 4 560 312 A2

## Description

**Field**

The present disclosure concerns methods of determining mechanical properties of rock samples, methods of determining values of anisotropy parameters from rock samples, and methods of determining parameters indicative of the porosity, bulk density or matrix density of rock samples, as well as associated computer programs, computer-readable media, data carrier signals and data sets, and methods of compiling associated data sets for use in such methods.

**Background**

Rock mechanics is the study of the mechanical behaviour of rocks, i.e. the mechanical response of rocks to applied forces. Knowledge of the mechanical properties of subsurface rock strata is useful in hydrocarbon exploration because such properties determine the rock's response to both natural environmental and artificially applied forces. For example, the mechanical properties of subsurface rocks can be used in the development and interpretation of seismic models, for calculating rock strength, and for determining the pressure environments required to fracture rock or to maintain rock fractures. An understanding of the fracture behaviour of subsurface sedimentary rocks is especially useful in unconventional hydrocarbon exploration (for example, for hydraulic fracturing of rocks in lateral hydrocarbon wells). Improved methods for determining the mechanical properties of rocks would therefore be of benefit.

**Summary**

According to a first aspect, a method comprises determining a mechanical property of a rock sample.
The method may comprise taking into account (a) a respective amount of each of two or more constituent phases in the rock sample and (b) a corresponding mechanical property parameter associated with each of the two or more constituent phases.
It may be that determining the mechanical property of the rock sample comprises evaluating a weighted sum of the mechanical property parameters associated with each of the two or more constituent phases. The weighted sum may be a weighted average of the mechanical property parameters associated with each of the two or more constituent phases. The mechanical property parameter associated with each constituent phase may be weighted in the weighted sum (e.g. weighted average) by the amount of said constituent phase in the rock sample.
The method may comprise determining (for example, estimating or measuring (e.g. directly or indirectly)) the respective amount of the two or more constituent phases. Additionally, the method may comprise determining (e.g. calculating) the mechanical property of the rock sample taking into account (a) the respective determined (e.g. estimated or measured) amount of each of the two or more constituent phases and (b) the corresponding mechanical property parameter associated with each of the two or more constituent phases.
It will be appreciated that each constituent phase is substantially chemically and/or structurally distinct. Each constituent phase is also typically substantially chemically and/or structurally homogeneous. Nevertheless, each constituent phase need not be a single material but may be a composite of two or more materials (i.e. together forming a composite phase). Each constituent phase is not necessarily located in one (e.g. contiguous) region of the rock sample but may be distributed across a plurality of (e.g. separate) regions of the rock sample. For example, it may be that different regions of a first constituent phase in the rock sample are spaced apart from one another by one or more second constituent phases in the rock sample.
It will further be appreciated that the method does not necessarily comprise determining and/or taking into account the amount of all constituent phases in the rock sample. Instead, the two or more constituent phases determined and/or taken into account may be a subset of the total number of constituent phases in the rock sample. For example, it may be that one or more minor constituent phases in the rock sample are present in negligible amounts and, therefore, it is not necessary to take into account the presence of the one or more minor constituent phases in the rock sample when determining the mechanical property of the rock sample. However, the two or more constituent phases determined and/or taken into account may comprise (e.g. be) a majority of the constituent phases in the rock sample. For example, the two or more constituent phases determined and/or taken into account may together constitute no less than about 80 %, for example, no less than about 90 %, or no less than about 95 %, or no less than about 99 %, of the total volume of the rock. Nevertheless, in some examples, the two or more constituent phases determined and/or taken into account constitute all of the constituent phases in the rock sample.
It may be that determining the respective amount of each of the two or more constituent phases in the rock sample comprises determining the amount of at least one constituent phase (for example, at least two, or at least three, or the majority of, or all of the constituent phases) in the rock sample by a spectroscopic method. For example, the spectroscopic method may be an infra-red spectroscopic method. For example, the spectroscopic method may be Fourier Transform Infra-red (FTIR) spectroscopy.

It may be that determining the amount of the at least one constituent phase (for example, the at least two, or the at least three, or the majority of, or all of the constituent phases) in the rock sample by the spectroscopic method (e.g. by the infra-red spectroscopic method, for example by Fourier Transform Infra-red (FTIR) spectroscopy) comprises: obtaining a spectroscopic measurement from the rock sample; and determining the amount of the at least one constituent phase (for example, the at least two, or the at least three, or the majority of, or all of the constituent phases) in the rock sample based on the spectroscopic measurement and a spectroscopic calibration model which defines a relationship between spectroscopic measurements and constituent phase amounts for rock samples.

The spectroscopic measurement may comprise (e.g. be) a value of a spectroscopic parameter (for example, an emission or absorption signal (e.g. intensity) at a particular wavelength) or a plurality of values of a spectroscopic parameters (for example, emission or absorption signals (e.g. intensities) at a plurality of different wavelengths), e.g. a spectroscopic (emission or absorption) spectrum.

The spectroscopic calibration model may define a mathematical relationship (e.g. a functional relationship or mapping) between the spectroscopic measurements and the constituent phase amounts for rock samples. The spectroscopic calibration model may therefore be (or be represented by) a mathematical function. The mathematical function may be expressed (or expressable) in an analytical or a numerical form. The mathematical function may be parameterised based on (i.e. in terms of) spectroscopic calibration model parametrisation data, for example as stored in a look-up table.

At least one constituent phase (e.g. determined by the spectroscopic method) may be a solid constituent phase (it being understood that, in embodiments in which more than one constituent phase is determined by the spectroscopic method, the at least two, or the at least three, or the majority of or all of the constituent phases are typically different solid constituent phases). Each solid constituent phase may be a mineralogical phase (for example, a mineral phase or a mineraloid phase) or an organic phase (i.e. a solid or substantially solid (i.e. semi-solid) organic phase such as kerogen, bitumen or pyrobitumen). Each solid constituent phase may be a single material (e.g. a single mineral or mineraloid) or a composite comprising two or more materials (e.g. a composite comprising two or more minerals and/or mineraloids). Example minerals include quartz, feldspar, calcite, dolomite, pyrite and clay minerals (such as kaolinite, illite and montmorillonite). Example mineraloids include opal and obsidian. An example composite phase is a mineral matrix phase comprising two or more minerals and/or mineraloids. Example organic phases include kerogen, bitumen and pyrobitumen.

It may be that determining the respective amount of each of the two or more constituent phases in the rock sample comprises: determining a parameter indicative of the porosity of the rock sample (e.g. determining the porosity of the rock sample); and determining the amount of at least one constituent phase in the rock sample based on the parameter indicative of the porosity of the rock sample (e.g. based on the porosity of the rock sample).

The at least one constituent phase in the rock sample (i.e. determined based on the parameter indicative of the porosity of the rock sample) may be at least one liquid phase in the rock sample. For example, the at least one liquid phase may be a hydrocarbon phase (i.e. a liquid hydrocarbon phase). Alternatively, the at least one liquid phase may be an aqueous phase (i.e. a liquid aqueous phase) such as water.

The method may comprise determining the parameter indicative of the porosity of the rock sample (e.g. determining the porosity of the rock sample) by a water immersion porosimetry (WIP) method. For example, the method may comprise determining the parameter indicative of the porosity of the rock sample (e.g. determining the porosity of the rock sample) by the methods according the fourteenth or seventeenth aspects described in more detail hereinbelow.

The rock sample may be a core sample. The skilled person will appreciate that a core sample is a cylindrical section of rock having standardised dimensions. For example, a core sample may be a cylindrical section of rock having a diameter of about 1 inch. Plugs may be extracted from core samples for detailed analysis.

Alternatively, the rock sample may be a cuttings sample. The skilled person will appreciate that a cuttings sample is a sample of drill cuttings obtained when a well is drilled. Drill cuttings typically comprise (e.g. consist of) relatively small, broken pieces of rock produced by drilling action and brought to the surface in drilling mud. Cuttings samples are commonly examined as part of mud logging (i.e. well logging) processes.

It may be that the amount of each constituent phase in the rock sample is a parameter indicative of a volume (e.g. total volume) of the said constituent phase in the rock sample. The parameter indicative of a volume (e.g. total volume) of the said constituent phase in the rock sample may be a volume (e.g. total volume) of the said constituent phase in the rock sample. Alternatively, the parameter indicative of a volume of the said constituent phase in the rock sample may be a volume fraction of the said constituent phase in the rock sample (e.g. the fraction of the total volume of the rock sample constituted by the said constituent phase). Accordingly, it may be that determining the mechanical property of the rock sample comprises evaluating a volume-weighted sum (e.g. volume-weighted average) of the mechanical property parameters associated with each of the two or more constituent phases, wherein the mechanical property parameter associated with each constituent phase is weighted in the volume-weighted sum (e.g. volume-weighted average) by the parameter indicative of the volume of (e.g. the volume (e.g. total volume) of or the volume fraction of) said constituent phase in the rock sample.

Alternatively, it may be that the amount of each constituent phase in the rock sample is a parameter indicative of a mass (e.g. total mass) of the said constituent phase in the rock sample. The parameter indicative of a mass (e.g. total mass) of the said constituent phase in the rock sample may be a mass (e.g. total mass) of the said constituent phase in the rock sample. Alternatively, the parameter indicative of a mass of the said constituent phase in the rock sample may be a mass fraction of the said constituent phase in the rock sample (e.g. the fraction of the total mass of the rock sample constituted by the said constituent phase). Accordingly, it may be that determining the mechanical property of the rock sample comprises evaluating a mass-weighted sum (e.g. mass-weighted average) of the mechanical property parameters associated with each of the two or more constituent phases, wherein the mechanical property parameter associated with each constituent phase is weighted in the mass-weighted sum (e.g. mass-weighted average) by the parameter indicative of the mass of (e.g. the mass (e.g. total mass) of or the mass fraction of) said constituent phase in the rock sample.

The mechanical property of the rock sample may be a static mechanical property of the rock sample, such as an elastic modulus of the rock sample or a dimensionless mechanical property ratio (i.e. such that the method may be a method of determining a static mechanical property such as an elastic modulus or a dimensionless mechanical property ratio of the rock sample). The elastic modulus may be the Young's modulus of the rock sample (i.e. a measure of the resistance of the rock sample to elastic deformation under uniaxial stretching or compression, i.e. the stiffness of the rock sample). The elastic modulus may be the shear modulus of the rock sample (i.e. a measure of the resistance of the rock sample to elastic shear strain). The elastic modulus may be the bulk modulus of the rock sample (i.e. a measure of the resistance of the rock sample to elastic deformation under hydrostatic pressure). The dimensionless mechanical property ratio may be the Poisson's ratio of the rock sample (i.e. the ratio of the lateral and longitudinal strains induced in the rock sample when it is subjected to uniaxial tensile stress).

Alternatively, the mechanical property of the rock sample may be a dynamic mechanical property of the rock sample such as such a velocity of a (e.g. S-mode or P-mode) acoustic wave or a (e.g. S-mode or P-mode) acoustic wave travel time (i.e. DTS ("delta-transit-time shear") or DTC ("delta-transit-time compressional")) in the rock sample (for example, in a particular direction).

In embodiments in which determining the mechanical property of the rock sample comprises evaluating a weighted sum (e.g. weighted average) of the mechanical property parameters associated with each of the two or more constituent phases, the mechanical property parameters associated with each of the two or more constituent phases may be static mechanical property parameters such as elastic modulus parameters, Young's modulus parameters, shear modulus parameters, bulk modulus parameters or dimensionless mechanical property ratio parameters such as Poisson's ratio parameters, dependent on the mechanical property to be determined.

Alternatively, the mechanical property parameters associated with each of the two or more constituent phases may be dynamic mechanical property parameters such as velocities of (e.g. S-mode or P-mode) acoustic waves or (e.g. S-mode or P-mode) acoustic wave travel times (i.e. DTS or DTC) in each of the two or more constituent phases. For example, the method may comprise determining the dynamic mechanical property of the rock sample by evaluating a weighted sum (e.g. weighted average) of the dynamic mechanical property parameters associated with each of the two or more constituent phases.

In some examples, the method comprises determining a static mechanical property of the rock sample by: determining one or more dynamic mechanical properties of the rock sample; and determining (e.g. calculating) a static mechanical property of the rock sample based on the one or more determined dynamic mechanical properties. For example, the method may comprise: determining the one or more dynamic mechanical properties of the rock sample by evaluating one or more weighted sums of dynamic mechanical property parameters associated with each of the two or more constituent phases; and determining (e.g. calculating) the static mechanical property of the rock sample based on the one or more determined dynamic mechanical properties. For example, the method may comprise: determining S-mode and P-mode acoustic wave travel times (i.e. DTS or DTC) (or, equivalently, determining velocities of S-mode and P-mode acoustic waves) in the rock sample (for example, by evaluating a weighted sum (e.g. weighted average) of S-mode acoustic wave travel times (i.e. DTS) associated with each of the two or more constituent phases and by evaluating a weighted sum (e.g. weighted average) of P-mode acoustic wave travel times (i.e. DTC) associated with each of the two or more constituent phases (or, equivalently, by evaluating a weighted sum (e.g. weighted average) of velocities of S-mode acoustic waves associated with each of the two or more constituent phases and by evaluating a weighted sum (e.g. weighted average) of velocities of P-mode acoustic waves associated with each of the two or more constituent phases)); and determining (e.g. calculating) one or more static mechanical properties (e.g. the shear modulus, Young's modulus, bulk modulus and/or Poisson's ratio) of the rock sample based on the one or more determined dynamic mechanical properties.

One or more (e.g. all) of the mechanical property parameters (e.g. static mechanical property parameters and/or dynamic mechanical property parameters) associated with each of the two or more constituent phases may be (or may be calculated based on) known reference values, for example stored in a look-up table. The known reference values may be derived from, for example, experimental measurements of the mechanical properties of constituent phases or estimated mechanical properties (e.g. based on *ab initio* or phenomenological calculations) for constituent phases.

Additionally or alternatively, the method may further comprise determining one or more (e.g. all) of the mechanical property parameters (e.g. static mechanical property parameters and/or dynamic mechanical property parameters) associated with one or more of the two or more constituent phases. For example, a constituent phase may be a composite of two or more materials. The method may therefore comprise determining a mechanical property parameter of the composite, for example taking into account (a) a respective amount of each of the two or more materials in the composite and (b) a corresponding mechanical property parameter associated with each of the two or more materials. It may be that determining the mechanical property parameter of the composite comprises evaluating a weighted sum (e.g. weighted average) of the mechanical property parameters associated with each of the two or more materials, wherein the mechanical property parameter associated with each material may be weighted in the weighted sum (e.g. weighted average) by the amount of said material in the composite. An example constituent phase comprising two or more materials is a rock mineral matrix which may comprise two or more minerals and/or mineraloids. The rock mineral matrix may comprise a plurality of crystals or grains of the two or more minerals and/or mineraloids which are randomly oriented with respect to one another such that the rock mineral matrix may be considered to be a single (although composite) phase.

In some examples, the rock sample is a shale rock sample. In examples in which the rock sample is a shale rock sample, the method may comprise determining a dynamic mechanical property of the rock sample by evaluating a weighted sum (e.g. weighted average) of dynamic mechanical property parameters associated with a mineral matrix phase, a clay mineral phase, an organic phase and a liquid phase in the rock sample. The method may comprise: determining the dynamic mechanical property of the rock sample by evaluating the weighted sum (e.g. weighted average) of dynamic mechanical property parameters associated with the mineral matrix phase, the clay mineral phase, the organic phase and the liquid phase in the rock sample; and determining (e.g. calculating) a static mechanical property of the rock sample based on the determined dynamic mechanical property. The method may comprise determining the dynamic mechanical property parameter of the mineral matrix phase by evaluating a weighted sum (e.g. weighted average) of dynamic mechanical property parameters associated with two or more minerals and/or mineraloids in the mineral matrix phase. Additionally or alternatively, the method may comprise determining (e.g. calculating) the dynamic mechanical property parameter of the clay mineral phase based on known reference values (for example, based on known reference values for one or more coefficients of elasticity for the clay mineral phase). Additionally or alternatively, the method may comprise determining (e.g. calculating) the dynamic mechanical property parameter of the organic phase based on known reference values (for example, based on known reference values for one or more elastic moduli for the organic phase). Additionally or alternatively, the method may comprise determining the dynamic mechanical property parameter of the liquid phase based on known reference values.

In some examples, the method comprises determining the mechanical property (e.g. the static mechanical property or the dynamic mechanical property) of the rock sample along a particular direction. For example, the rock sample may be a sample of a rock having an axis of symmetry, wherein mechanical properties of the rock are isotropic within planes perpendicular to the axis of symmetry but vary along the axis of symmetry (i.e. such that the rock can be considered a vertically transverse isotropic (VTI) medium). The method may comprise determining the mechanical property (e.g. the static mechanical property or the dynamic mechanical property) of the rock sample in a direction parallel to the axis of symmetry (i.e. in a 'vertical' direction) or in a direction perpendicular to the axis of symmetry (i.e. in a 'horizontal' direction). Accordingly, one or more of the mechanical property parameters taken into account in determining the mechanical property of the rock sample may be directional mechanical property parameters, wherein a directional mechanical property parameter is a mechanical property parameter of the constituent phase in a particular direction (e.g. in the vertical direction or the horizontal direction, as appropriate). The method may comprise taking into account directional mechanical property parameters for anisotropic constituent phases (e.g. clay mineral phases), where present, and non-directional mechanical property parameters (i.e. isotropic mechanical property parameters) for isotropic constituent phases (e.g. mineral matrix phases), where present. Additionally or alternatively, the method may comprise taking into account non-directional mechanical property parameters (i.e. isotropic mechanical property parameters or, for example, averaged mechanical property parameters, wherein an averaged mechanical property parameter for a given phase is obtained by averaging a directional mechanical property parameter for the given phase in the vertical direction and a corresponding directional mechanical property parameter for the said phase in the horizontal direction) for one or more anisotropic constituent phases. The method may use a non-directional mechanical property parameter for an anisotropic constituent phase when the effects of mechanical property anisotropy in the said constituent phase are averaged out across a rock sample, for example due to a random distribution of grains within the rock sample. An example rock which may be considered to be a VTI medium is a shale rock.

The method may further comprise taking into account an anisotropy factor associated with (e.g. of) the rock sample when determining the mechanical property. For example, the method may comprise determining the mechanical property of the rock sample by evaluating a weighted sum (e.g. weighted average) of the mechanical property parameters associated with each of the two or more constituent phases taking into account the anisotropy factor.

The method may further comprise determining (e.g. measuring) the anisotropy factor associated with (e.g. of) the rock sample. For example, the method may comprise determining (e.g. measuring) the anisotropy factor associated with (e.g. of) the rock sample by the method according to the fifth aspect as described in more detail hereinbelow.

It may be that the anisotropy factor is a mechanical anisotropy factor. The mechanical anisotropy factor may be indicative of anisotropy in one or more mechanical properties of the rock sample. The mechanical anisotropy factor may be determined (e.g. measured) by mechanical testing methods, for example by mechanical indentation-based testing methods.

Alternatively, it may be that the anisotropy factor is a magnetic anisotropy factor. The magnetic anisotropy factor may be indicative of the anisotropy in one or more magnetic properties of the rock sample. The magnetic anisotropy factor may be determined (e.g. measured) by magnetic testing methods, for example by magnetic susceptibility testing methods, for example by anhysteric magnetic susceptibility testing methods.

It may be that one or more steps of the method are carried out by a computer. For example, it may be that the method comprises the computer determining the mechanical property of the rock sample. It may be that the method comprises the computer taking into account (a) the respective amount of each of two or more constituent phases in the rock sample and (b) the corresponding mechanical property parameter associated with each of the two or more constituent phases. It may be that the method comprises the computer evaluating the weighted sum (e.g. weighted average) of the mechanical property parameters associated with each of the two or more constituent phases.

In a second aspect, a computer program comprises instructions which, when the program is executed by a computer, cause the computer to carry out one or more steps of the method according to the first aspect. For example, it may be that the instructions, when the program is executed by the computer, cause the computer to carry out any combination of the steps of the method of the first aspect identified hereinabove as being carried out by (or being suitable for being carried out by) a computer.

In a third aspect, there is provided a (e.g. non-transitory) computer-readable medium storing the computer program (e.g. the instructions) according to the second aspect. The computer program (e.g. the instructions) may be stored as computer-executable program code.

In a fourth aspect, there is provided a data carrier signal carrying (e.g. encoding) the computer program (e.g. the instructions) according to the second aspect. The computer program (e.g. the instructions) may be provided in the form of computer-executable program code.

In a fifth aspect, a method comprises determining (e.g. measuring) a value of an anisotropy parameter indicative of anisotropy in a rock sample. The method may comprise determining (e.g. measuring) the value of the anisotropy parameter based on a spectroscopic measurement obtained from the rock sample (e.g. using a spectroscopic method).

It may be that the spectroscopic measurement is obtained by infra-red spectroscopy (i.e. an infra-red spectroscopic method). It may be that the spectroscopic measurement is obtained by Fourier Transform Infra-red (FTIR) spectroscopy (i.e. an FTIR spectroscopic method).

It may be that determining (e.g. measuring) the value of the anisotropy parameter comprises: obtaining a spectroscopic measurement from the rock sample (e.g. by the infra-red spectroscopic method, for example by FTIR spectroscopy); and determining the value of the anisotropy parameter based on the spectroscopic measurement and a spectroscopic calibration model which defines a relationship between spectroscopic measurements and the anisotropy parameter for rock samples.

The spectroscopic measurement may comprise (e.g. be) a value of a spectroscopic parameter (for example, an emission or absorption signal (e.g. intensity) at a particular wavelength) or a plurality of values of a spectroscopic parameters (for example, emission or absorption signals (e.g. intensities) at a plurality of different wavelengths), e.g. a spectroscopic (emission or absorption) spectrum.

The spectroscopic calibration model may define a mathematical relationship (e.g. a functional relationship or mapping) between the spectroscopic measurements and the anisotropy parameter for rock samples. The spectroscopic calibration model may therefore be (or be represented by) a mathematical function. The mathematical function may be expressed (or expressable) in an analytical form or a numerical form. The mathematical function may be parameterised based on (i.e. in terms of) spectroscopic calibration model parametrisation data, for example stored in a look-up table.

The anisotropy parameter may be a mechanical anisotropy parameter. The mechanical anisotropy parameter may be indicative of mechanical anisotropy in the rock sample. The spectroscopic calibration model may define a relationship (e.g. mapping) between spectroscopic measurements and mechanical anisotropy parameter measurements for rock samples. The mechanical anisotropy parameter measurements may be mechanical anisotropy parameter measurements obtained using mechanical indentation-based measurement methods.

Alternatively, the anisotropy parameter may be a magnetic anisotropy parameter. The magnetic anisotropy parameter may be indicative of magnetic anisotropy in the rock sample. The spectroscopic calibration model may define a relationship (e.g. mapping) between spectroscopic measurements and magnetic anisotropy parameter measurements for rock samples. The magnetic anisotropy parameter measurements may be magnetic anisotropy parameter measurements obtained using magnetic susceptibility measurement methods such as anhysteric magnetic susceptibility measurement methods.

The rock sample may be a core sample. Alternatively, the rock sample may be a cuttings sample.

It may be that one or more steps of the method are carried out by a computer. For example, it may be that the method comprises the computer determining the value of the anisotropy parameter based on a spectroscopic measurement obtained from the rock sample. It may be that the method comprises the computer determining the value of anisotropy parameter based on a spectroscopic measurement and the spectroscopic calibration model.

In a sixth aspect, a computer program comprises instructions which, when the program is executed by a computer, cause the computer to carry out one or more steps of the method according to the fifth aspect. For example, it may be that the instructions, when the program is executed by the computer, cause the computer to carry out any combination of the steps of the method of the fifth aspect identified hereinabove as being carried out by (or being suitable for being carried out by) a computer.

In a seventh aspect, there is provided a (e.g. non-transitory) computer-readable medium storing the computer program (e.g. the instructions) according to the sixth aspect. The computer program (e.g. the instructions) may be stored as computer-executable program code.

In an eighth aspect, there is provided a data carrier signal carrying (e.g. encoding) the computer program (e.g. the instructions) according to the sixth aspect. The computer program (e.g. the instructions) may be provided in the form of computer-executable program code.

In a ninth aspect, a method comprises fitting a spectroscopic calibration model to spectroscopic measurement data and corresponding anisotropic parameter measurement data obtained from a plurality of (i.e. different) reference rock samples, wherein the spectroscopic calibration model defines a relationship (e.g. a mathematical or functional relationship, e.g. a mapping) between the spectroscopic measurement data and the corresponding anisotropic parameter measurement data for the plurality of (i.e. different) reference rock samples.

Accordingly, it may be that the method comprises: providing the spectroscopic measurement data obtained from the plurality of (i.e. different) reference rock samples; and providing the corresponding anisotropic parameter measurement data obtained from the (i.e. same) plurality of (i.e. different) reference rock samples. The method may further comprise: obtaining the spectroscopic measurement data from the plurality of (i.e. different) reference rock samples (i.e. using a spectroscopic method); and obtaining the corresponding anisotropic parameter measurement data from the (i.e. same) plurality of (i.e. different) reference rock samples (i.e. using a spectroscopic method).

The spectroscopic measurement data may be infra-red spectroscopic measurement data. For example, the spectroscopic measurement data may be Fourier Transform Infra-red (FTIR) spectroscopic measurement data.

The anisotropic parameter measurement data may be mechanical anisotropic parameter measurement data. For example, the anisotropic parameter measurement data may be mechanical indentation-based anisotropic parameter measurement data.

Alternatively, the anisotropic parameter measurement data may be magnetic anisotropic parameter measurement data. For example, the anisotropic parameter measurement data may be magnetic susceptibility-based magnetic anisotropic parameter measurement data such as anhysteric magnetic susceptibility-based magnetic anisotropic parameter measurement data.

One or more steps of the method may be carried out by a computer. For example, the method may comprise the computer fitting the spectroscopic calibration model to the spectroscopic measurement data and the corresponding anisotropic parameter measurement data. The spectroscopic calibration model may be fitted to the spectroscopic measurement data and the corresponding anisotropic parameter measurement data using a multivariate statistical method such as a least squares regression method. However, the skilled person will appreciate that any other suitable fitting methods as known in the art may be used. For example, the spectroscopic calibration model may be fitted to the spectroscopic measurement data and the corresponding anisotropic parameter measurement data using a machine learning algorithm (for example, using a neural network or a genetic algorithm) as known in the art.

In a tenth aspect, a computer program comprises instructions which, when the program is executed by a computer, cause the computer to carry out one or more steps of the method according to the ninth aspect. For example, it may be that the instructions, when the program is executed by the computer, cause the computer to carry out any combination of the steps of the method of the ninth aspect identified hereinabove as being carried out by (or being suitable for being carried out by) a computer.

In an eleventh aspect, there is provided a data set comprising the spectroscopic measurement data of the ninth aspect and/or the anisotropic parameter measurement data of the ninth aspect and/or spectroscopic calibration model parameter data on which (i.e. in terms of which) the spectroscopic calibration model of the ninth aspect is parametrised.

In a twelfth aspect, there is provided a (e.g. non-transitory) computer-readable medium storing the computer program (e.g. the instructions) according to the tenth aspect and/or the data set according to the eleventh aspect. The computer program (e.g. the instructions) may be stored as computer-executable program code.

In a thirteenth aspect, there is provided a data carrier signal carrying (e.g. encoding) the computer program (e.g. the instructions) according to the tenth aspect and/or the data set according to the eleventh aspect. The computer program (e.g. the instructions) may be provided in the form of computer-executable program code.

In a fourteenth aspect, a method of determining a parameter indicative of a porosity of a rock sample comprises:

measuring a mass of the rock sample when the rock sample is saturated by and submerged in a liquid, thereby obtaining the saturated, submerged mass of the rock sample; measuring a mass of the rock sample in air when the rock sample is saturated by the liquid, thereby obtaining the saturated mass of the rock sample in air; measuring a mass of the rock sample in air when the rock sample is dry, thereby obtaining the dry mass of the rock sample in air; and calculating the parameter indicative of the porosity of the rock sample based on the saturated, submerged mass of the rock sample, the saturated mass of the rock sample in air, the dry mass of the rock sample in air and the density of the liquid.

The parameter indicative of the porosity of the rock sample may be the porosity of the rock sample. The porosity, $\phi$, of the rock sample may be determined according to

$$\phi = \frac{\rho_M - \rho_B}{\rho_M - \rho_L},$$

wherein $\rho_M$ is the rock matrix material density, $\rho_B$ is the sample bulk density and $\rho_L$ is the density of the liquid.

The method may be a method of determining the parameter indicative of the porosity of the rock sample by immersion porosimetry, for example by water immersion porosimetry (WIP). The liquid may, therefore, be water.

The rock sample may be a core sample. Alternatively, the rock sample may be a cuttings sample.

The method may comprise, prior to measuring the mass of the rock sample (i.e. prior to obtaining the saturated, submerged mass of the rock sample, the saturated mass of the rock sample in air, and the dry mass of the rock sample in air), washing the rock sample. Washing the rock sample may comprise washing the rock sample in water and detergent. Additionally or alternatively, washing the rock sample may comprise sonicating the rock sample, for example sonicating the rock sample in water. Washing the rock sample may comprise: washing the rock sample in water and detergent; and subsequently sonicating the rock sample, for example in water. Washing the rock sample may comprise washing the rock sample in water and detergent and/or sonicating the rock sample in water until the water runs clear (e.g. for a minimum washing time period, such as at least 10 minutes of washing or at least 20 minutes of washing).

In an fifteenth aspect, a method of determining a parameter indicative of a bulk density of a rock sample comprises: measuring a mass of the rock sample when the rock sample is saturated by and submerged in a liquid, thereby obtaining the saturated, submerged mass of the rock sample; measuring a mass of the rock sample in air when the rock sample is saturated by the liquid, thereby obtaining the saturated mass of the rock sample in air; measuring a mass of the rock sample in air when the rock sample is dry, thereby obtaining the dry mass of the rock sample in air; and calculating the parameter indicative of the bulk density of the rock sample based on the saturated, submerged mass of the rock sample, the saturated mass of the rock sample in air, the dry mass of the rock sample in air and the density of the liquid.

The parameter indicative of the bulk density of the rock sample may be the bulk density of the rock sample. The bulk density, $\rho_B$, of the rock sample may be determined according to

$$\rho_B = \frac{m_{\text{Sat, Air}}\rho_L}{m_{\text{Dry, Air}} - m_{\text{Sat, Sub}}},$$

wherein $m_{\text{Sat, Sub}}$ is the saturated, submerged mass of the rock sample, $m_{\text{Sat, Air}}$ is the saturated mass of the rock sample in air, $m_{\text{Dry, Air}}$ is the dry mass of the rock sample in air, and $\rho_L$ is the density of the liquid.

The method may be a method of determining the parameter indicative of the bulk density of the rock sample by immersion porosimetry, for example by water immersion porosimetry (WIP). The liquid may, therefore, be water.

The rock sample may be a core sample. Alternatively, the rock sample may be a cuttings sample.

The method may comprise, prior to measuring the mass of the rock sample (i.e. prior to obtaining the saturated, submerged mass of the rock sample, the saturated mass of the rock sample in air, and the dry mass of the rock sample in air), washing the rock sample. Washing the rock sample may comprise washing the rock sample in water and detergent. Additionally or alternatively, washing the rock sample may comprise sonicating the rock sample, for example sonicating the rock sample in water. Washing the rock sample may comprise: washing the rock sample in water and detergent; and subsequently sonicating the rock sample, for example in water. Washing the rock sample may comprise washing the rock sample in water and detergent and/or sonicating the rock sample in water until the water runs clear (e.g. for a minimum washing time period, such as at least 10 minutes of washing or at least 20 minutes of washing).

In a sixteenth aspect, a method of determining a parameter indicative of a matrix density of a rock sample comprises: measuring a mass of the rock sample when the rock sample is saturated by and submerged in a liquid, thereby obtaining the saturated, submerged mass of the rock sample; measuring a mass of the rock sample in air when the rock sample is dry, thereby obtaining the dry mass of the rock sample in air; and calculating the parameter indicative of the matrix density of the rock sample based on the saturated, submerged mass of the rock sample and the dry mass of the rock sample in air.

The parameter indicative of the matrix density of the rock sample may be the matrix density of the rock sample.

The matrix density, $\rho_M$, of the rock sample may be determined according to

$$\rho_M = \frac{m_{\text{Dry, Air}}}{m_{\text{Dry, Air}} - m_{\text{Sat, Sub}}},$$

wherein $m_{\text{Sat, Sub}}$ is the saturated, submerged mass of the rock sample and $m_{\text{Dry, Air}}$ is the dry mass of the rock sample in air.

The method may be a method of determining the parameter indicative of the matrix density of the rock sample by immersion porosimetry, for example by water immersion porosimetry (WIP). The liquid may, therefore, be water.

The rock sample may be a core sample. Alternatively, the rock sample may be a cuttings sample.

The method may comprise, prior to measuring the mass of the rock sample (i.e. prior to obtaining the saturated, submerged mass of the rock sample and the dry mass of the rock sample in air), washing the rock sample. Washing the rock sample may comprise washing the rock sample in water and detergent. Additionally or alternatively, washing the rock sample may comprise sonicating the rock sample, for example sonicating the rock sample in water. Washing the rock sample may comprise: washing the rock sample in water and detergent; and subsequently sonicating the rock sample, for example in water. Washing the rock sample may comprise washing the rock sample in water and detergent and/or sonicating the rock sample in water until the water runs clear (e.g. for a minimum washing period, such as at least 10 minutes of washing or at least 20 minutes of washing).

In a seventeenth aspect, a method comprises determining a matrix density of a rock sample taking into account (a) a respective amount of each of two or more constituent phases in the rock sample and (b) a corresponding density of each of the two or more constituent phases.

It may be that determining the matrix density of the rock sample comprises evaluating a weighted sum (e.g. weighted average) of the densities of each of the two or more constituent phases. The density of each constituent phase may be weighted in the weighted sum (e.g. weighted average) by the amount of said constituent phase in the rock sample.

The method may comprise determining (for example, estimating or measuring (e.g. directly or indirectly)) the respective amount of the two or more constituent phases in the rock sample. Additionally, the method may comprise determining (e.g. calculating) the matrix density of the rock sample taking into account (a) the respective determined (e.g. estimated or measured) amount of each of the two or more constituent phases and (b) the corresponding density of each of the two or more constituent phases.

It will be appreciated that the method does not necessarily comprise determining and/or taking into account the amount of all constituent phases in the rock sample. Instead, the two or more constituent phases determined and/or taken into account may be a subset of the total number of constituent phases in the rock sample. For example, it may be that one or more minor constituent phases in the rock sample are present in negligible amounts and, therefore, it is not necessary to take into account the presence of the one or more minor constituent phases in the rock sample when determining the matrix density of the rock sample. However, the two or more constituent phases determined and/or taken into account may comprise (e.g. be) a majority of the constituent phases in the rock sample. For example, the two or more constituent phases determined and/or taken into account may together constitute no less than about 80 %, for example, no less than about 90 %, or no less than about 95 %, or no less than about 99 %, of the total volume of the rock. Nevertheless, in some examples, the two or more constituent phases determined and/or taken into account constitute all of the constituent phases in the rock sample.

It may be that determining the respective amount of each of the two or more constituent phases in the rock sample comprises determining the amount of at least one constituent phase (for example, at least two, or at least three, or the majority of, or all of the constituent phases) in the rock sample by a spectroscopic method. For example, the spectroscopic method may be an infra-red spectroscopic method. For example, the spectroscopic method may be Fourier Transform Infra-red (FTIR) spectroscopy.

It may be that determining the respective amount of each of the two or more constituent phases in the rock sample by the spectroscopic method (e.g. by the infra-red spectroscopic method, for example by Fourier Transform Infra-red (FTIR) spectroscopy) comprises: obtaining a spectroscopic measurement from the rock sample; and determining the amount of said constituent phase in the rock sample based on the spectroscopic measurement and a spectroscopic calibration model which defines a relationship between spectroscopic measurements and constituent phase amounts for rock samples.

The spectroscopic measurement may comprise (e.g. be) a value of a spectroscopic parameter (for example, an emission or absorption signal (e.g. intensity) at a particular wavelength) or a plurality of values of a spectroscopic parameter (for example, emission or absorption signals (e.g. intensities) at a plurality of different wavelengths, e.g. a spectroscopic (emission or absorption) spectrum.

The spectroscopic calibration model may define a mathematical relationship (e.g. a functional relationship or mapping) between the spectroscopic measurements and the constituent phase amounts for rock samples. The spectroscopic calibration model may therefore be (or be represented as) a mathematical function. The mathematical function may be expressed (or expressible) in an analytical or a numerical form. The mathematical function may be parameterised based on (i.e. in terms of) spectroscopic calibration model parametrisation data, for example stored in a look-up table.

The at least one constituent phase (i.e. determined by the spectroscopic method) may be a solid constituent phase (it being understood that, in embodiments in which more than one constituent phase is determined by the spectroscopic method, the at least two, or the at least three, or the majority of or all of the constituent phases are typically different solid constituent phases). Each solid constituent phase may be a mineralogical phase (for example, a mineral phase or a mineraloid phase) or an organic phase (i.e. a solid or substantially solid (i.e. semi-solid) organic phase such as kerogen, bitumen or pyrobitumen). Each solid constituent phase may be a single material (e.g. a single mineral or mineraloid) or a composite comprising two or more materials (e.g. a composite comprising two or more minerals and/or mineraloids). Example minerals include quartz, feldspar, calcite, dolomite, pyrite and clay minerals (such as kaolinite, illite and montmorillonite). Example mineraloids include opal and obsidian. Example organic phases include kerogen, bitumen and pyrobitumen.

It may be that the amount of each constituent phase in the rock sample is a parameter indicative of a volume (e.g. total volume) of the said constituent phase in the rock sample. The parameter indicative of a volume (e.g. total volume) of the said constituent phase in the rock sample may be a volume (e.g. total volume) of the said constituent phase in the rock sample. Alternatively, the parameter indicative of a volume of the said constituent phase in the rock sample may be a volume fraction of the said constituent phase in the rock sample (e.g. the fraction of the total volume of the rock sample constituted by the said constituent phase). Accordingly, it may be that determining the matrix density of the rock sample comprises evaluating a volume-weighted sum (e.g. volume-weighted average) of the densities of each of the two or more constituent phases, wherein the matrix density of each constituent phase is weighted in the volume-weighted sum (e.g. volume-weighted average) by the parameter indicative of the volume of (e.g. the volume (e.g. total volume) of or the volume fraction of) said constituent phase in the rock sample.

Alternatively, it may be that the amount of each constituent phase in the rock sample is a parameter indicative of a mass (e.g. total mass) of the said constituent phase in the rock sample. The parameter indicative of a mass (e.g. total mass) of the said constituent phase in the rock sample may be a mass (e.g. total mass) of the said constituent phase in the rock sample. Alternatively, the parameter indicative of a mass of the said constituent phase in the rock sample may be a mass fraction of the said constituent phase in the rock sample (e.g. the fraction of the total mass of the rock sample constituted by the said constituent phase). Accordingly, it may be that determining the matrix density of the rock sample comprises evaluating a mass-weighted sum (e.g. mass-weighted average) of the densities of each of the two or more constituent phases, wherein the density of each constituent phase is weighted in the mass-weighted sum (e.g. mass-weighted average) by the parameter indicative of the mass of (e.g. the mass (e.g. total mass) of or the mass fraction of) said constituent phase in the rock sample.

It will be appreciated that the density of a constituent phase is the mass density of said constituent phase, i.e. the mass per unit volume of the constituent phase. It will further be appreciated that the matrix density of a rock sample is the mass density (i.e. the mass per unit volume) of the rock sample taking into account only the solid material present in the rock sample (i.e. excluding the contribution of any pores or other voids in the rock sample to the total volume of the rock sample and excluding the contribution of any liquid filling such pores or voids to the total mass of the rock sample).

The rock sample may be a core sample. Alternatively, the rock sample may be a cuttings sample.

The method may further comprise determining (e.g. calculating) a parameter indicative of the porosity of the rock sample (e.g. calculating the porosity of the rock sample) based on the determined matrix density of the rock sample (e.g. and based on the bulk density of the rock sample, for example as determined according to the fifteenth aspect described hereinabove).

The method may be carried out by a computer. For example, the method may comprise the computer determining the matrix density of a rock sample taking into account (a) the respective amount of each of two or more constituent phases in the rock sample and (b) the corresponding density of each of the two or more constituent phases. The method may comprise the computer evaluating the weighted sum (e.g. weighted average) of the densities of each of the two or more constituent phases.

In an eighteenth aspect, a computer program comprises instructions which, when the program is executed by a computer, cause the computer to carry out one or more steps of the method according to the seventeenth aspect. For example, it may be that the instructions, when the program is executed by the computer, cause the computer to carry out any combination of the steps of the method of the seventeenth aspect identified hereinabove as being carried out by (or being suitable for being carried out by) a computer.

In a nineteenth aspect, there is provided a (e.g. non-transitory) computer-readable medium storing the computer program (e.g. the instructions) according to the eighteenth aspect. The computer program (e.g. the instructions) may be stored as computer-executable program code.

In a twentieth aspect, there is provided a data carrier signal carrying (e.g. encoding) the computer program (e.g. the instructions) according to the eighteenth aspect. The computer program (e.g. the instructions) may be provided in the form of computer-executable program code.

The skilled person will appreciate that, except where mutually exclusive, a feature described in relation to any one of the above aspects may be applied *mutatis mutandis* to any other aspect. Furthermore, except where mutually exclusive, any feature described herein may be applied to any aspect and/or combined with any other feature described herein.

**Figures**

Embodiments will now be described by way of example only, with reference to the Figures, in which:

**Figure 1** illustrates an oil shale rock formation imaged at six different length scales by optical photography, computerised tomography (CT), μCT, scanning electron microscopy (SEM), focused ion beam SEM, and ultra-high-resolution SEM;

**Figure 2** is a table of density and mechanical property parameter values for common rock matrix mineral phases;

**Figure 3** is a table of density and mechanical property parameter values for common rock clay mineral phases;

**Figure 4** is a table of density and mechanical property parameter values for two different types of rock organic matter phases;

**Figure 5** is a table of density and mechanical property parameter values for water and a liquid hydrocarbon commonly found in oil shales;

**Figure 6** is a plot comparing values of bulk density obtained for the same rock samples using a standard water immersion porosimetry (WIP) method developed for core samples and a new WIP method developed for cuttings samples;

**Figure 7** is an example FTIR absorption spectrum obtained from a rock sample;

**Figure 8** is a plot comparing measurements of the weight fraction of quartz in the same rock samples using quantitative X-ray diffraction (QXRD) and calibrated Fourier-transform infra-red spectroscopy (FTIR);

**Figure 9** is a plot comparing measurements of the weight fraction of feldspar in the same rock samples using quantitative X-ray diffraction (QXRD) and calibrated Fourier-transform infra-red spectroscopy (FTIR);

**Figure 10** is a plot comparing measurements of the weight fraction of carbonate in the same rock samples using quantitative X-ray diffraction (QXRD) and calibrated Fourier-transform infra-red spectroscopy (FTIR);

**Figure 11** is a plot comparing measurements of the weight fraction of I+S Group in the same rock samples using quantitative X-ray diffraction (QXRD) and calibrated Fourier-transform infra-red spectroscopy (FTIR);

**Figure 12** is a plot comparing measurements of the weight fraction of ankerite in the same rock samples using quantitative X-ray diffraction (QXRD) and calibrated Fourier-transform infra-red spectroscopy (FTIR);

**Figure 13** is a plot comparing measurements of the weight fraction of total organic carbon (TOC) in the same rock samples using quantitative X-ray diffraction (QXRD) and calibrated Fourier-transform infra-red spectroscopy (FTIR);

**Figure 14** is a plot comparing measurements of the S1 Rock-Eval parameter for the same rock samples using Rock-Eval and calibrated Fourier-transform infra-red spectroscopy (FTIR);

**Figure 15** is a plot comparing measurements of the S2 Rock-Eval parameter for the same rock samples using Rock-Eval and calibrated Fourier-transform infra-red spectroscopy (FTIR);

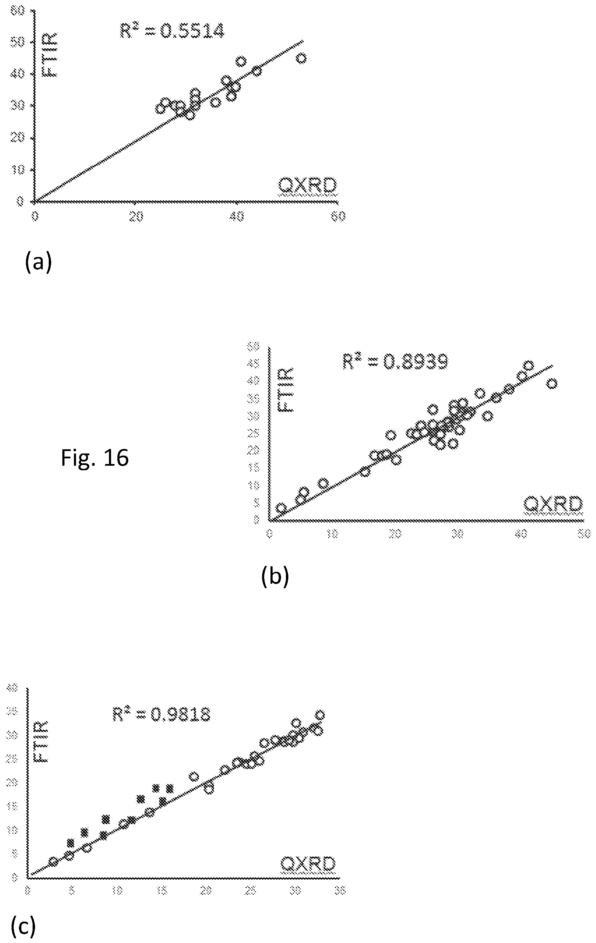

**Figure 16** shows three plots comparing measurements of a parameter in the same rock samples using QXRD and calibrated FTIR, the number of samples tested increasing from (a) to (c);

**Figure 17** is a plot comparing values of porosity determined for the same rock samples using a WIP method and a calibrated FTIR method;

**Figure 18** shows four plots comparing values of DTC, DTS, Young's modulus (E) and Poisson's ratio as a function of depth in a hydrocarbon well as determined using standard mechanical property testing methodologies (filled squares) and by calculation based on QXRD and WIP data (filled circles connected by solid lines);

**Figure 19** shows four plots comparing values of DTC, DTS, Young's modulus (E) and Poisson's ratio as a function of depth in a hydrocarbon well as determined using wirelines measurements and by calculation based on QXRD and WIP data;

**Figure 20** shows two plots comparing calculated and directly measured values of Young's modulus in the vertical and horizontal directions for a plurality of different shale rock samples;

**Figure 21** shows two plots comparing calculated and directly measured values of Poisson's ratio in the vertical and horizontal directions for a plurality of different shale rock samples;

**Figure 22** shows two plots comparing values of Young's modulus and Poisson's ratio determined for the same core samples using standard static mechanical property testing methods and by calculation based on dynamic ultrasonic measurements of acoustic wave travel times;

**Figure 23** is a plot comparing values of Young's modulus determined for the same rock samples using standard mechanical property testing methods ("Corelab data") and by calculation based on calibrated FTIR measurements ("Modelled data");

**Figure 24(a)** shows a plot comparing values of Poisson's ration determined for the same rock samples using dynamic ultrasonic measurements of acoustic wave travel times ("Dynamic measured") and by calculation based on calibrated FTIR measurements ("Modelled Poisson's ratio");

**Figure 24(b)** shows the same results as Figure 24 (a) following removal of anisotropy from the calculation based on calibrated FTIR;

**Figure 25** shows a plot comparing values of an anisotropy parameter determined for the same rock samples using nanoindentation and calculation based on calibrated FTIR measurements ("Modelled anisotropy)";

**Figure 26** shows a computer processor in communication with a computer-readable medium storing a computer program comprising computer-executable instructions; and

**Figure 27** shows a plot comparing values of static Young's modulus in horizontal and vertical directions obtained by standard rock mechanic measurements from multiple samples taken from an unconventional basin from multiple stratigraphic intervals and locations (line A labelled with circles), and for two different sets of core samples taken from north and south of the basin (line B labelled with triangles and line C labelled with squares).

**Detailed description**

*Rock mechanics*

Rock mechanics is the study of the mechanical behaviour of rocks and rocks masses, i.e. the mechanical response of rocks to applied forces. Of particular concern in hydrocarbon exploration is the determination of the mechanical properties of subsurface rocks and, in particular, of subsurface sedimentary rock strata, which determine the rock's response to both natural environmental and artificially applied forces.

For example, geophysicists use the mechanical properties of subsurface rocks in the development and interpretation of seismic models. Knowledge of the mechanical properties of subsurface rocks is also required for accurate calculations of rock strength and of the pressure environments required to fracture rock (e.g., by hydraulic fracturing) or to maintain rock fractures (whether man-made or naturally occurring). Drilling engineers may also use knowledge of rock properties to avoid accidental fracture of rocks (for example, to reduce the risk of a blowout in an overpressured formation).

As discussed in more detail below, Young's modulus and Poisson's ratio are two important rock parameters used in the design, formation and maintenance of wells used in hydrocarbon exploration. Moreover, knowledge of the mechanical properties of layered or laminated rocks, such as shales, is of particular importance because the majority of economically significant hydrocarbon reservoirs are located in sedimentary basins. An understanding of the fracture behaviour of subsurface sedimentary rocks is especially useful in unconventional hydrocarbon exploration (for example, for hydraulic fracturing of rocks in lateral wells). For example, Young's modulus can be used to calculate the fracture width which can be achieved using hydraulic fracturing processes, and the fracture width correlates with the hydrocarbon production achievable in a well.

The mechanical properties of rocks extracted from hydrocarbon wells can be measured precisely in the laboratory, for example using indentation-based mechanical testing techniques. Such mechanical testing methods typically require the use of core samples. A core sample is a cylindrical section of rock having standardised dimensions. It can, however, be difficult and expensive to obtain core samples for analysis. For example, it may not be possible to extract core samples from a sufficient number of different sampling locations along a lateral hydrocarbon well to obtain an accurate picture of the variation in rock mechanical properties along the well. The mechanical testing of core samples by standard laboratory methods can also be also time-consuming.

One alternative to the use of core samples is to carry out the required laboratory measurements on cuttings samples. Cuttings samples are samples of the drill cuttings obtained when a well is drilled; drill cuttings are typically small, broken pieces of rock produced by the drilling action and brought to the surface in the drilling mud. Cuttings samples are therefore typically plentiful, as well as easy and inexpensive to obtain. Cuttings are typically examined as part of mud logging (i.e., well logging) analysis, which includes observation of the cuttings, microscopic examination and basic chemical analysis. It is, however, generally understood that cuttings samples are not suitable for detailed mechanical property testing. For example, cuttings samples cannot be used with standardised indentation-based testing methods. Nevertheless, the present inventors have developed methods which can be used to obtain rock mechanical properties from cuttings samples, as set out in the following sections.

*Elastic constants*

The principal mechanical properties of concern in the present application are the elastic properties of rocks, that is to say, the properties (or parameters) of rocks which govern their elastic behaviour. The elastic properties of rocks, as with other materials more generally, can be quantified in terms of elastic constants.

It will be appreciated that the linearly elastic behaviour of a solid material is governed by Hooke's law, which can be expressed for a continuous elastic material as

$$\boldsymbol{\sigma} = -\boldsymbol{c}\boldsymbol{\varepsilon}, \tag{1}$$

where $\sigma$ is the second-order stress tensor and $\varepsilon$ is the second-order strain tensor, defined by

$$\boldsymbol{\sigma} = \begin{bmatrix} \sigma_{11} & \sigma_{12} & \sigma_{13} \\ \sigma_{21} & \sigma_{22} & \sigma_{23} \\ \sigma_{31} & \sigma_{32} & \sigma_{33} \end{bmatrix} \tag{2}$$

And

$$\boldsymbol{\varepsilon} = \begin{bmatrix} \varepsilon_{11} & \varepsilon_{12} & \varepsilon_{13} \\ \varepsilon_{21} & \varepsilon_{22} & \varepsilon_{23} \\ \varepsilon_{31} & \varepsilon_{32} & \varepsilon_{33} \end{bmatrix}, \tag{3}$$

and c is the fourth-order stiffness or elasticity tensor which provides a linear map between stress and strain. Hooke's law can therefore be written in Einstein notation as

$$\sigma_{ij} = -c_{ijkl}\varepsilon_{kl}. \tag{4}$$

Although the stiffness tensor c contains 81 elements $c_{ijkl}$ (otherwise known as coefficients of elasticity), only 21 of these elements are independent due to the inherent symmetries of $\sigma$, **c** and $\varepsilon$, even in the most general anisotropic material. Accordingly, Hooke's law can be rewritten in terms of the independent coefficients of elasticity, in matrix notation, as

$$\begin{bmatrix} \sigma_{11} \\ \sigma_{22} \\ \sigma_{33} \\ \sigma_{23} \\ \sigma_{13} \\ \sigma_{12} \end{bmatrix} = \begin{bmatrix} c_{1111} & c_{1122} & c_{1133} & c_{1123} & c_{1113} & c_{1112} \\ & c_{2222} & c_{2233} & c_{2223} & c_{2213} & c_{2212} \\ & & c_{3333} & c_{3323} & c_{3313} & c_{3312} \\ & & & c_{2323} & c_{2313} & c_{2312} \\ & \text{symm} & & & c_{1313} & c_{1312} \\ & & & & & c_{1212} \end{bmatrix} \begin{bmatrix} \varepsilon_{11} \\ \varepsilon_{22} \\ \varepsilon_{33} \\ 2\varepsilon_{23} \\ 2\varepsilon_{13} \\ 2\varepsilon_{12} \end{bmatrix} . \tag{5}$$

Utilising the symmetries of $\sigma$, $\mathbf{c}$ and $\varepsilon$, Hooke's law can be further simplified by writing in the "engineering" or Voigt notation as

$$\begin{bmatrix} \sigma_1 \\ \sigma_2 \\ \sigma_3 \\ \sigma_4 \\ \sigma_5 \\ \sigma_6 \end{bmatrix} = \begin{bmatrix} c_{11} & c_{12} & c_{13} & c_{14} & c_{15} & c_{16} \\ & c_{22} & c_{23} & c_{24} & c_{25} & c_{26} \\ & & c_{33} & c_{34} & c_{35} & c_{36} \\ & & & c_{44} & c_{45} & c_{46} \\ & \text{symm} & & & c_{55} & c_{56} \\ & & & & & c_{66} \end{bmatrix} \begin{bmatrix} \varepsilon_1 \\ \varepsilon_2 \\ \varepsilon_3 \\ \varepsilon_4 \\ \varepsilon_5 \\ \varepsilon_6 \end{bmatrix} , \tag{6}$$

where pairs of indices are transformed according to the following rule

$$( \ )_{11} \rightarrow ( \ )_1$$
$$( \ )_{22} \rightarrow ( \ )_2$$
$$( \ )_{33} \rightarrow ( \ )_3$$
$$( \ )_{23} \rightarrow ( \ )_4 \tag{7}$$
$$( \ )_{13} \rightarrow ( \ )_5$$
$$( \ )_{12} \rightarrow ( \ )_6$$

and engineering shear strains are defined by the sum of symmetric shear components, such as

$$\varepsilon_4 = 2\varepsilon_{23} = \varepsilon_{23} + \varepsilon_{32}. \tag{8}$$

The number of coefficients is, however, reduced further in systems having particular symmetries. For example, while triclinic systems have twenty-one independent coefficients, monoclinic systems have thirteen, orthotropic systems have nine, transverse isotropic systems have five, and cubic systems have three. In fact, there are only two independent elastic coefficients in an isotropic medium (i.e. a medium having the same physical properties in all directions).

The coefficients of elasticity, $c_{ijkl}$ (or $c_{ij}$ in the Voigt notation), are also related to the engineering constants (also known as 'elastic constants' or 'elastic moduli') which can be obtained directly from experimental measurements. For example, for an isotropic medium, one finds from experiment that the components of the strain tensor can be expressed as

$$\varepsilon_{11} = \frac{1}{E}\big(\sigma_{11} - \nu(\sigma_{22} + \sigma_{33})\big), \tag{9}$$

$$\varepsilon_{22} = \frac{1}{E}\big(\sigma_{22} - \nu(\sigma_{11} + \sigma_{33})\big), \tag{10}$$

$$\varepsilon_{33} = \frac{1}{E}\big(\sigma_{33} - \nu(\sigma_{11} + \sigma_{22})\big), \tag{11}$$

$$2\varepsilon_{23} = \frac{\sigma_{23}}{G}, \tag{12}$$

$$2\varepsilon_{13} = \frac{\sigma_{13}}{G}, \tag{13}$$

And

$$2\varepsilon_{12} = \frac{\sigma_{12}}{G}, \tag{14}$$

where $E$ is Young's modulus, v is Poisson's ratio and G is the engineering shear modulus. Young's modulus is a measure of the resistance of a material to elastic deformation under uniaxial stretching or compression (i.e. the stiffness of the material). Poisson's ratio is the ratio of the lateral and longitudinal strains induced in a material subjected to uniaxial tensile stress. The shear modulus is a measure of the resistance of a material to elastic shear strains.

These engineering constants are related to one another according to

$$G = \frac{E}{2(1+v)}. \tag{15}$$

The engineering constants are also related to the Lamé parameters by

$$\mu = G = \frac{E}{2(1+v)} \tag{16}$$

and

$$\lambda = \frac{Ev}{(1+v)(1-2v)}, \tag{17}$$

where Hooke's law for an isotropic material can be expressed as

$$\sigma_{ij} = 2\mu\varepsilon_{ij} + \lambda\delta_{ij}\varepsilon_{kk} \tag{18}$$

and $\delta_{ij}$ is the Kronecker delta.

The engineering constants $E$, $v$ and $G$ are also related to the bulk modulus, $K$, (which is a measure of the response of the material to hydrostatic pressure) by

$$K = \frac{E}{3(1-v)}. \tag{19}$$

**Accordingly,** due to its inherent symmetries, the mechanical behaviour of an isotropic medium can be predicted using measured values of any two of $E$, v, $G$ and $K$.

In geology, the elastic constants defined so far are commonly referred to as *static* elastic constants. Static elastic constants are those constants obtained through standard laboratory stress-strain measurements such as tensile testing or mechanical indentation. It is, however, also possible to derive *dynamic* elastic constants for materials. Dynamic elastic constants are determined based on measurements of the velocity at which sound waves travel through a material. Dynamic elastic constants may be measured in the laboratory using very high (i.e., ultrasonic) frequencies. However, dynamic properties of subterranean rocks may also be obtained through analysis of low-frequency sonic log data captured *in situ.*

The velocity of sound in a given material depends on the material's mechanical properties (i.e. the coefficients of elasticity or, at least for an isotropic medium, $E$, v, $G$ and/or $K$). Sound can also be propagated through a material in different modes. Two of the most important modes for the analysis of rock behaviour are the compressional (P) and shear (S) modes.

The compressional sound wave velocity, $v_P$, and the shear sound wave velocity, $v_S$, can be expressed in terms of the compressional wave travel time, DTC (i.e. " delta-transit-time compressional"), and the shear wave travel time, DTS (i.e. "delta-transit-time shear"), obtained experimentally from seismic measurements or sonic testing, as

$$v_P = \frac{10^6}{\text{DTC}} \qquad\qquad (20)$$

And

$$v_S = \frac{10^6}{\text{DTS}}. \qquad\qquad (21)$$

The compressional and shear wave travel times, DTC and DTS, are typically measured in units of $\mu$s/m (i.e. microseconds per metre) or $\mu$s/ft (i.e. microseconds per foot), while the compressional and shear wave velocities, $v_P$ and $v_S$, are measured in units of m/s (i.e. metres per second) or ft/s (i.e. feet per second), with the factor of $10^6$ accounting for the conversion between microseconds and seconds.

Because the propagation of sounds waves through a material depends on the elastic behaviour of that material, the following relationships between the elastic constants, on the one hand, and the sound wave travel times, on the other hand, can be derived for an isotropic material:

$$G = A\,\frac{\rho}{\text{DTS}^2}, \qquad\qquad (22)$$

$$K = A\rho\left(\frac{1}{\text{DTC}^2} - \frac{4}{3\text{DTS}^2}\right), \qquad\qquad (23)$$

$$\nu = \frac{0.5\left(\frac{\text{DTS}}{\text{DTC}}\right)^2 - 1}{\left(\frac{DTS}{DTC}\right)^2 - 1} \qquad\qquad (24)$$

And

$$E = 2G(1 + \nu), \qquad\qquad (25)$$

where $\rho$ is the density of the material and $A$ is a coefficient equal to 1000 (in metric units) or 13400 (in imperial units) based on the above definitions of $v_P$ and $v_S$.

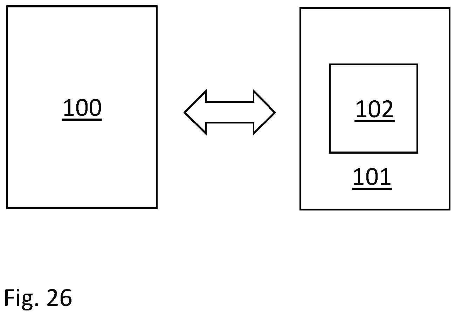

Dynamic measurements of DTS and DTC can, therefore, be used to determine the static elastic constants $E$, $\nu$, $G$ and $K$. The skilled person will appreciate that such calculations are suited to automation and implementation in computer software (for example, computer software 102 stored on a computer-readable medium 101, for execution by a computer processor 100, as shown in Figure 26).

*Rocks*

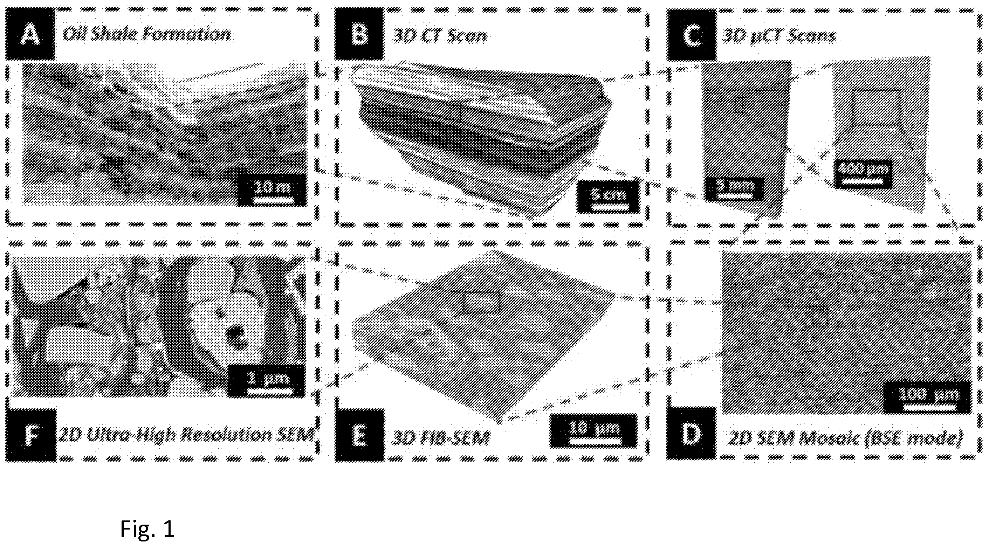

The expressions of the previous section were derived for isotropic media. However, many properties of rocks are not, in general, isotropic. Indeed, many types of rock exhibit marked anisotropy on different length scales. Sedimentary rocks, in particular, exhibit a structural layering across multiple length scales which results in mechanical property anisotropy. For example, Figure 1 illustrates structural anisotropy in shale rocks from the level of rock formations (visible to the naked eye) down to sub-micron length scales (observable in an ultra-high-resolution scanning electron microscope (SEM)). Accordingly, inaccuracies may occur when expressions for the elastic constants of isotropic media are used in predicting the mechanical behaviour of rocks.

Nevertheless, geologists have found that the mechanical behaviour of many rock types can be approximated by models which account for anisotropy in a relatively simple way. For example, sedimentary rocks (such as shales) can be modelled as vertically transverse isotropic (VTI) media, with good agreement between predicted properties and experimental results. A VTI medium (also known as a transversely isotropic medium, a polar anisotropic medium or a radially anisotropic medium) has physical properties which are symmetric about an axis (commonly referred to as the 'vertical' axis, it being understood that 'vertical' in this context does not necessarily imply a particular orientation of the rock) normal to a transverse plane of isotropy. Accordingly, the physical properties of the VTI medium are isotropic (i.e. the same in all directions) *within* a plane of isotropy, but said properties vary *between* parallel planes.

Rocks are, in general, also composite materials. That is to say, rocks are not chemically or structurally homogeneous materials, but are instead aggregates of different mineral or mineraloid (i.e. non-crystalline mineral-like substances such as obsidian) phases having different chemical compositions and structures. The mechanical properties of rocks, therefore, depend on the particular rock constituent phases and their relative arrangements.

For example, a shale rock typically comprises a plurality of layered clay (i.e. silicate) mineral sheets held together by a chemically and structurally distinct mineral matrix. The mineral matrix typically comprises a mixture of randomly oriented matrix mineral crystals, such as crystals of quartz, feldspar, calcite, dolomite, pyrite, etc. Shale rock may also include organic material, such as kerogen, bitumen and pyrobitumen.

Rocks are also commonly porous. For example, shale rocks may contain pores which are filled with fluid, such as water and/or hydrocarbons. The existence of pores within the rock changes the overall rock mechanical properties. The speed at which sound waves are transmitted through a rock also depends on the level of porosity and the nature of any fluid filling the pores.

The present inventors have found that estimates of rock mechanical properties can be obtained using cuttings samples by considering the detailed composite nature of rock. In particular, the velocity of an acoustic wave in either of the P or S modes in the shale rock can be expressed in terms of a volume-weighted average as

$$v_{\mathrm{Rock}} = V_A v_A + V_B v_B + V_C v_C + \cdots, \qquad (26)$$

where $v_X$ and $V_X$ are, respectively, the acoustic wave velocity in, and the volume fraction of, a given constituent component (i.e., phase) X in the rock, and the sum is taken over all such components in the rock. Similarly, the respective acoustic wave travel times, DTS and DTC, can also be determined as a volume-weight average according to

$$\mathrm{DTS}_{\mathrm{Rock}} = V_A \mathrm{DTS}_A + V_B \mathrm{DTS}_B + V_C \mathrm{DTS}_C + \cdots \qquad (27)$$

And

$$\mathrm{DTC}_{\mathrm{Rock}} = V_A \mathrm{DTC}_A + V_B \mathrm{DTC}_B + V_C \mathrm{DTC}_C + \cdots, \qquad (28)$$

where $\mathrm{DTS}_X$ and $\mathrm{DTC}_X$ are, respectively, the partial acoustic wave travel times, DTS and DTC, for a given constituent component (i.e., phase) X in the rock, and the sum is taken over all such components in the rock. The skilled person will appreciate that such calculations are suited to automation and implementation in computer software (for example, computer software 102 stored on a computer-readable medium 101, for execution by a computer processor 100, as shown in Figure 26).

The static elastic constants of the rock can be determined, as discussed hereinabove, from the acoustic velocities and travel times.

Accordingly, in order to determine the static elastic constants of a rock based on a cuttings sample, it is necessary to determine the volume fraction of each phase present in the sample, as well as the acoustic wave velocities and travel times for each said phase. In general, the acoustic wave velocities and travel times for the most commonly encountered rock constituent phases are already known or can be determined readily based on known experimental data (as discussed in more detailed below in relation to shale rocks in particular). Such properties can also be measured experimentally, for example on the basis of core samples. Moreover, the volume fraction of each phase present in a cuttings sample can be determined experimentally. This may be achieved using, for example, X-ray diffraction-based methods, which are accurate, although relatively time-consuming. The present inventors have, however, also developed combined experimental and modelling methods which enable rapid determination of the volume fraction of the various phases present in a rock cuttings sample using spectroscopic techniques, as discussed in more detail in subsequent sections.

The calculation of rock mechanical properties using Equations (22) to (28) will now be discussed in more detail with particular reference to shale rocks.

*Shale rocks*

The mechanical properties of a shale rock depend generally on: the properties of the clay (i.e. silicate) mineral sheets, the matrix minerals and the organic material which make up the rock, as well as the properties of any fluids present in pores; the relative amounts of each of these components; and, potentially, the structural arrangement of the various components within the rock structure.

Applying Equations (26) to (28), therefore, the velocity of an acoustic wave in either of the P or S modes in the shale rock can be expressed as

$$v_{\text{Rock}} = V_{\text{Matrix}} v_{\text{Matrix}} + V_{\text{Clay}} v_{\text{Clay}} + V_{\text{Organic}} v_{\text{Organic}} + V_{\text{Pore}} v_{\text{Pore}}, \qquad (29)$$

where $v_{\text{Matrix}}$ is the acoustic wave velocity in the mineral matrix, $v_{\text{Clay}}$ is the acoustic wave velocity in the clay sheets, $v_{\text{Organic}}$ is the acoustic wave velocity in the organic material, $v_{\text{Pore}}$ is the acoustic wave velocity in the (typically fluid-filled) pores, and $V_{\text{X}}$ is again the corresponding volume fraction of the rock made up of each component X. The respective acoustic wave travel times, DTS and DTC, can also be determined as

$$\text{DTS}_{\text{Rock}} = V_{\text{Matrix}} \text{DTS}_{\text{Matrix}} + V_{\text{Clay}} \text{DTS}_{\text{Clay}} + V_{\text{Organic}} \text{DTS}_{\text{Organic}} + V_{\text{Pore}} \text{DTS}_{\text{Pore}} \qquad (30)$$

and

$$\text{DTC}_{\text{Rock}} = V_{\text{Matrix}} \text{DTC}_{\text{Matrix}} + V_{\text{Clay}} \text{DTC}_{\text{Clay}} + V_{\text{Organic}} \text{DTC}_{\text{Organic}} + V_{\text{Pore}} \text{DTC}_{\text{Pore}}. \qquad (31)$$

Each of these components will now be considered in turn.

*Matrix minerals*

The elastic properties of the individual matrix minerals in a shale rock will, in general, be anisotropic. However, due to the large number of randomly oriented mineral grains typically present in the rock, it can generally be assumed that anisotropies in the elastic properties will average out across the grains and, therefore, the mineral matrix can be approximated as an isotropic material.

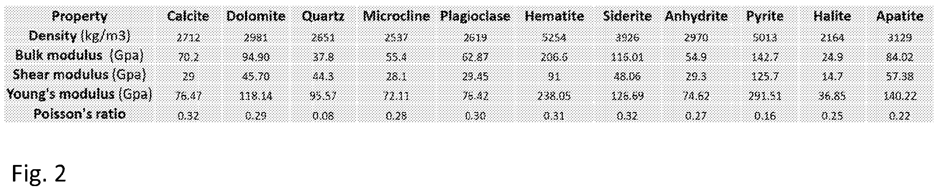

The elastic constants (e.g., $E, K,$ G and v) and the density ($\rho$) of the most common matrix minerals are well known (see, for example, Figure 2). Accordingly, the acoustic velocities and travel times for each individual mineral can be determined by applying Equations (20) to (25). Where the composition of the mineral matrix is known, the average values of DTS and DTC for the matrix, $\text{DTS}_{\text{Matrix}}$ and $\text{DTC}_{\text{Matrix}}$, can also be calculated again by multiplying the equivalent DTS or DTC value for a given mineral by the volume fraction of that mineral present in the matrix, i.e., such that:

$$\text{DTS}_{\text{Matrix}} = V_a \text{DTS}_a + V_b \text{DTS}_b + V_c \text{DTS}_c + \cdots, \qquad (32)$$

and

$$\text{DTC}_{\text{Matrix}} = V_a \text{DTC}_a + V_b \text{DTC}_b + V_c \text{DTC}_c + \cdots, \qquad (33)$$

where $V_x$ and $\text{DTS}_x$ are the volume fraction and DTS value for a mineral of type $x$ in the matrix, and the sum is taken over all mineral types present in the matrix.

In a similar way to the overall composition of the rock, the particular composition of the mineral matrix can be determined experimentally using, e.g., X-ray diffraction-based methods or spectroscopic techniques as discussed in more detail in subsequent sections.

*Clay sheets*

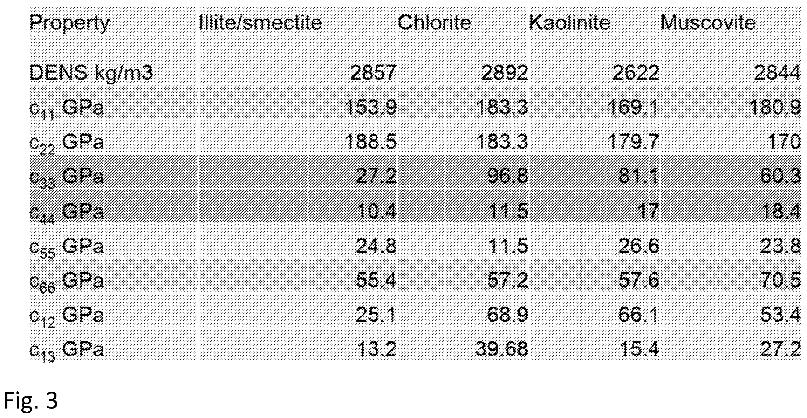

Due to the layered nature of clay minerals, they cannot be approximated accurately as isotropic components in the same way as the matrix minerals. Instead, the acoustic velocities and elastic constants of the clay sheets will depend on the orientation of the sheets in relation to the applied stress direction. However, since the clay mineral properties can be assumed to be isotropic *within* the individual sheets, it is only necessary to determine the elastic properties of the clay components in two principal directions: parallel (referred to as the 'horizontal' direction) and perpendicular (referred to as the 'vertical' direction) to the sheets. The coefficients of elasticity, $c_{ij}$ (in the Voigt notation), are known for the most common clay phases from experiment or *ab initio* calculations (see, for example, Figure 3, derived from Militzer, B, et al., First-principles calculation of the elastic moduli of sheet silicates and their application to shale anisotropy, American Mineralogist, 2011). Moreover, the velocities of P- and S-mode acoustic waves in both the vertical and horizontal directions can be expressed in terms of specific $c_{ij}$ elements according to:

$$v_{\text{P vertical}} = A\sqrt{\frac{c_{33}}{\rho}}, \tag{34}$$

$$v_{\text{P horizontal}} = A\sqrt{\frac{c_{11}}{\rho}}, \tag{35}$$

$$v_{\text{S vertical}} = A\sqrt{\frac{c_{44}}{\rho}}, \tag{36}$$

and

$$v_{\text{S horizontal}} = A\sqrt{\frac{c_{66}}{\rho}}, \tag{37}$$

where $A$ is again the coefficient equal to 1000 (in metric units) and $\rho$ is the grain density of the clay phase. The values of DTC and DTS for each mode and each direction can be determined from the corresponding values of $v_{\text{S horizontal}}$, $v_{\text{S vertical}}$, $v_{\text{P horizontal}}$, and $v_{\text{P vertical}}$.

*Organic material*

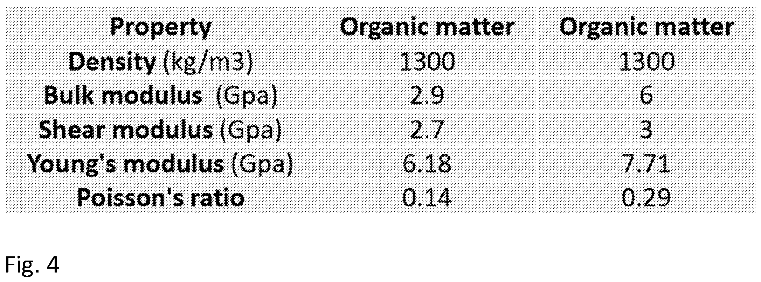

Organic materials such as kerogen, bitumen and pyrobitumen exhibit preferential alignment parallel to bedding planes in a similar way to the clay sheets. However, due to the typically variable composition of the organic materials found in rocks, it is difficult to determine precise values of the coefficients of elasticity, $c_{ij}$, which could be used to calculate the acoustic wave velocities. Nevertheless, acoustic wave velocities and travel times of organic phases can be back-calculated from known ranges of values (see, for example, Figure 4) for the bulk modulus, $K$, and the shear modulus, $G$, in the vertical and horizontal directions by applying Equations (20) to (23).
The total amount of organic material (often referred to as the amount of total organic carbon (TOC)) present in the rock can again be determined experimentally using, e.g., X-ray diffraction-based methods or spectroscopic techniques.

*Porosity*

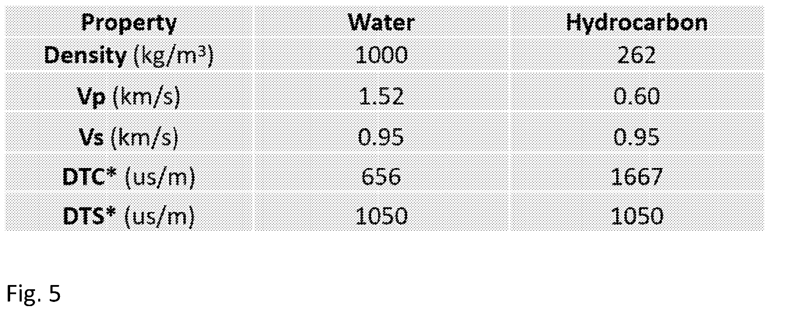

The contribution of the porous structure of shale rocks can be taken into account by assuming that the pores are filled entirely with fluid (i.e. liquid), which, lacking long-range structure, is isotropic. Values of $v_{\text{P}}$, $v_{\text{S}}$, DTC and DTS are known for the most common fluids found in petroliferous rocks, such as water or hydrocarbons (see, for example, Figure 5).
The total volume of the pores in the rock can be estimated or it can be measured directly. In this regard, the present inventors have developed new methods based on water immersion porosimetry (WIP) for determining the porosity, $\phi$, of a rock, defined by

$$\phi = \frac{\rho_M - \rho_B}{\rho_M - \rho_W}, \tag{38}$$

where $\rho_M$ is the rock matrix material density, $\rho_B$ is the sample bulk density and $\rho_W$ is the density of water.
In general, the rock matrix density of a sample can be determined experimentally by: first, determining which solid (or semi-solid) phases are present in the rock, as well as their respective volume fractions (for example, by using X-ray diffraction methods and Rock-Eval (i.e. Rock-Eval pyrolysis using a Rock-Eval analyser available from Vinci Technologies SA, Nanterre, France), LECO (i.e. LECO combustion analysis using a LECO carbon analyser available from the LECO Corporation, Saint Joseph, Michigan, USA) or calibrated spectroscopic methods, as discussed in more detail in subsequent sections); and second, summing the known densities for each of the phases present in the rock according to

$$\rho_M = V_a \rho_a + V_b \rho_b + V_c \rho_c + \cdots, \qquad (39)$$

where $V_x$ and $\rho_x$ are, respectively, the volume fraction and density for a constituent phase $x$ in the sample.

The bulk density of a sample can be determined by measuring the mass of the sample in air ($m_{Air}$) and the mass of the sample submerged in distilled water ($m_{Sub}$) and calculating $\rho_B$ according to

$$\rho_B = \frac{m_{Air} \rho_W}{m_{Air} - m_{Sub}}. \qquad (40)$$

Both $m_{Air}$ and $m_{Sub}$ can be determined by WIP using a balance provided with a platform for supporting the sample in air and a container, retaining distilled water, for measuring the sample in a submerged state. The apparatus may also be provided with a thermometer for measuring the temperature of the distilled water. A balance capable of measuring mass in grams to four decimal places (for example, as available from Mettler-Toledo Ltd., United Kingdom) has been found to be sufficient for determining $\rho_B$.

The porosity of a sample can then be calculated by applying Equation (38) to the values of $\rho_M$ and $\rho_B$ obtained from Equations (39) and (40). This methodology is suitable for determining the porosity of core samples.

However, the present inventors have now developed a WIP-based method for determining the porosity of *cuttings* samples. In this method, in general terms, the mass of a cuttings sample is measured under three different sets of conditions. First, the mass of the cuttings sample is measured while the sample is submerged in distilled water, resulting in a measurement of the saturated submerged mass ($m_{Sat,\ Sub}$). Second, the mass of the soaked cuttings sample is measured following air drying until surface water has evaporated from the sample, resulting in a measurement of saturated mass in air ($m_{Sat,\ Air}$). Third, the mass of the cuttings sample is measured following complete drying in an oven, resulting in a measurement of the dry mass in air ($m_{Dry,\ Air}$). The bulk density of the cuttings sample is then determined according to

$$\rho_B = \frac{m_{Sat,\ Air} \rho_W}{m_{Dry,\ Air} - m_{Sat,\ Sub}} \qquad (41)$$

and the matrix density is determined according to

$$\rho_M = \frac{m_{Dry,\ Air}}{m_{Dry,\ Air} - m_{Sat,\ Sub}}. \qquad (42)$$

The total porosity can then be calculated as usual according to Equation (38).

The inventors have also found that it is beneficial to wash the cuttings samples thoroughly prior to measurement of $m_{Sat,\ Sub}$, $m_{Sat,\ Air}$ and $m_{Dry,\ Air}$. An example washing protocol has two principal washing stages, which may be repeated as necessary. In a first washing stage, the cuttings samples are agitated in a container of warm water mixed with detergent. The cuttings samples are then further washed and agitated in a sieve under hot water. Additional detergent may be added and the cuttings samples are finally rinsed with warm water until the water is foam-free and clear. This first washing stage may be repeated until the samples appear clean under visual inspection. In a subsequent second washing stage, the cuttings samples are repeatedly washed and rinsed in a sieve with detergent and warm water until the water runs clear. The samples are then placed in a sonic bath filled with warm water and sonicated for 20 minutes. The samples are then rinsed in a sieve and then further sonicated in distilled water for 20 minutes. Sonication in distilled water is repeated as necessary until the water remains clear following 20 minutes of sonication.

Following washing, the samples may be dried and the matrix and bulk densities may be determined by the following example measurement protocol. First, the samples are dried overnight in an oven at between 50°C and 60°C. Following drying, the samples are placed in a pre-weighed aluminium cup and submerged in distilled water for a minimum of twelve hours, to ensure maximum possible saturation, prior to measurement of $m_{Sat,\ Sub}$. The samples are air-dried until surface water has evaporated prior to measurement of $m_{Sat,\ Air}$. The samples are then again dried overnight in an oven at between 50°C and 60°C prior to measurement of $m_{Dry,\ Air}$.

Mass measurements may be achieved using a similar experimental set up as described above with reference to measurement of the mass of core samples. In order to ensure that cuttings samples remain submerged during soaking and during submerged measurements, surfactant may be added to the distilled water to reduce its surface tension. A surfactant having a density equal to that of water may be used so as to avoid complicating the calculation of the porosity.

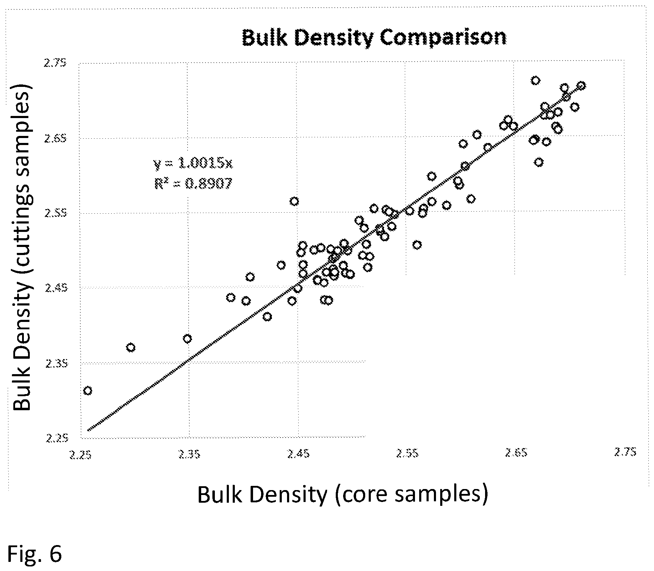

In order to assess the effectiveness of this new method, $\rho_B$ was measured by a standard WIP method for core samples and also by the new method for cuttings sample-sized chips taken from the core samples. The results are compared in Figure 6, which shows a good agreement between the results of both measurement techniques.

*Calibrated spectroscopy*

The present inventors have developed a spectroscopic method for determining the phase composition of cuttings samples. Compositions determined using this method can serve as input to the equations defined in the preceding sections for the calculation of dynamic properties such as acoustic wave velocities and travel times, as well as the rock matrix density, and therefore also the static elastic constants of rocks. While the skilled person will appreciate that the method may be implemented using many different kinds of spectroscopy, the discussion which follows is focused on the particular example of Fourier-transform infra-red spectroscopy (FTIR).

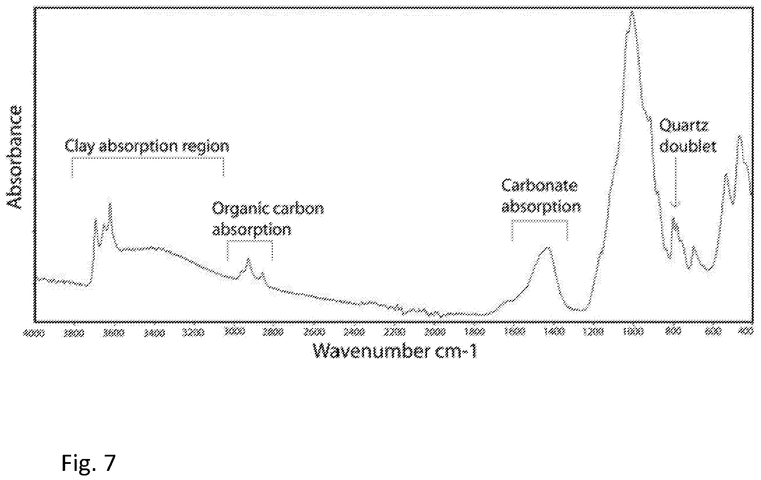

FTIR is a well-known spectroscopic technique for obtaining an infra-red absorption spectrum from a sample, the IR spectrum being reflective of the abundance of particular molecular bonds within the sample. FTIR typically makes use of attenuated total reflection (ATR). The sample (which may be powdered) is held in contact with an optically dense crystal having a high refractive index, and an infra-red beam is directed through the crystal at an angle sufficient to cause total internal reflection within the crystal, thereby generating an evanescent wave which extends beyond the surface of the crystal and into the sample. In regions of the infra-red spectrum where the sample absorbs energy, the evanescent wave will be attenuated or otherwise altered. Attenuated signal from each evanescent wave is passed back into the main infra-red beam which exits the crystal and is detected in the IR spectrometer, thereby generating an infra-red spectrum. For example, Figure 7 shows an example FTIR spectrum obtained from a rock sample, illustrating the regions of the spectrum associated with infra-red attenuation due to the presence of common mineralogical phases such as clays, quartz and carbonates, as well as TOC.

Although it is possible to identify mineralogical phases in spectra such as those obtained from FTIR (as seen in Figure 7), spectroscopic methods are not conventionally considered suitable for determining the mineral composition of a sample in a quantitative manner because the shape and height of the individual spectral peaks depend on interatomic bonding rather than mineralogy or crystal structure. However, the present inventors have found that quantitative spectroscopic mineralogical analysis of samples taken from a particular region is possible by fitting a spectroscopic model to a reference dataset compiled using more quantitatively accurate mineralogical analysis techniques, such as quantitative X-ray diffraction (QXRD), and a set of reference rock samples taken from the same region. The inventors have also found that quantitative measurements of carbon (e.g. TOC) content can also be achieved using a spectroscopic model fit to a reference dataset compiled using, for example, TOC combustion (e.g. LECO TOC) or pyrolysis (e.g. Rock-Eval) methods as applied to reference samples from the region.

In the new calibrated spectroscopic method developed by the inventors, a training data set of reference mineralogical and/or carbon content data is compiled using, e.g., QXRD, TOC combustion (e.g. LECO TOC) and/or pyrolysis (e.g. Rock-Eval) methods. QXRD can be used to determine the amounts of different phases present in multi-phase samples, as well as the characteristics of single phases, including the precise determination of crystal structure or crystallite size and shape. Quantitative XRD analysis requires the precise and accurate determination of the X-ray diffraction pattern of a sample, both in terms of peaks and intensities. The presence of organic phases in rock samples can also be determined using combustion or pyrolysis analysis methods (e.g. using a LECO instrument for combustion analysis or a Rock-Eval instrument for pyrolysis analysis). The reference training data set is compiled by measuring the mineralogical and/or carbon content data for a plurality of different samples of rock taken from an area. It has been found that a minimum of around 30 samples is typically required to build a representative training data set.

The reference training data set includes, for example, the amount (e.g. volume fraction) of each mineralogical phase and/or organic phase (e.g. TOC content) identified in each sample using each method. In addition, an FTIR spectrum (or other suitable spectroscopic spectrum) is obtained for each sample using an FTIR instrument (for example, an ALPHA FTIR spectrometer available from Bruker Corporation, Billerica, MA, United States of America) to compile an FTIR training data set. The FTIR training data set is then fit to the reference training data set using a multivariate statistical approach such as a least squares regression methodology (for example, using the OPUS spectroscopy software available from Bruker Corporation, Billerica, MA, United States of America). The FTIR spectra are matched to the mineralogical and organic components in the reference training data set and used to build a calibration model (also referred to as a chemometric model). In particular, multivariate calibration algorithms such as Partial Least Squares (PLS), as implemented in the OPUS spectroscopy software, can be used to correlate spectral intensity (e.g. absorbance values) in specified FTIR wavelength regions (i.e. peak areas in an FTIR spectrum) with concentration values for constituents in the reference training data set.

In the method developed by the inventors, cross-validation techniques are used to determine the quality of the calibration. Cross validation is a statistical process whereby a model is validated using the data points within it. For example, in a model fit to data obtained from 30 samples, cross validation could involve using the model to predict the results which would be expected for each of the 30 samples, one at a time, based on the data obtained from the other 29 samples in the set.

Once the calibration model has been built, it can be used to determine the mineral and/or organic content of an unknown rock sample based on a measured FTIR spectrum. This method is particularly suitable for the compositional analysis of large volumes of cuttings samples extracted from hydrocarbon wells, in particular due to the speed of the FTIR analysis and the subsequent comparison with the calibration model. For example, the inventors have found that the composition of a cuttings sample can be analysed within about 30 seconds, and an atmospheric calibration of only about 30 seconds is required between sequential sample analyses. Accordingly, the method is suited to the compositional analysis of cuttings samples from hydrocarbon wells during drilling.

In a variant of the method, an alert is triggered if a measured FTIR spectrum cannot be matched sufficiently accurately to the calibration model. Accuracy of a match can be assessed in terms of the distance of a measured FTIR spectrum from the calibration model, for example in terms of the Mahalanobis distance (which is a multidimensional generalisation of the method of using the number of standard deviations from the mean to quantify distance). If an alert is triggered, new reference mineralogical and/or organic content measurements are performed on the sample (i.e. using QXRD, TOC combustion (e.g. LECO TOC) and/or pyrolysis (e.g. Rock-Eval) methods, etc.) and the resultant data, as well as the measured FTIR spectrum, are incorporated into the existing calibration model, thereby extending the range of calibration. This enables the model to be developed and improved on-the-fly.

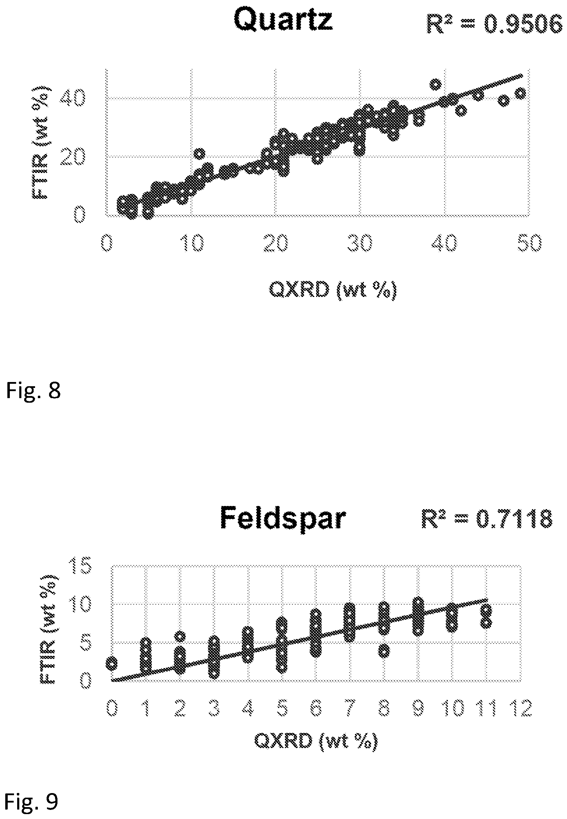

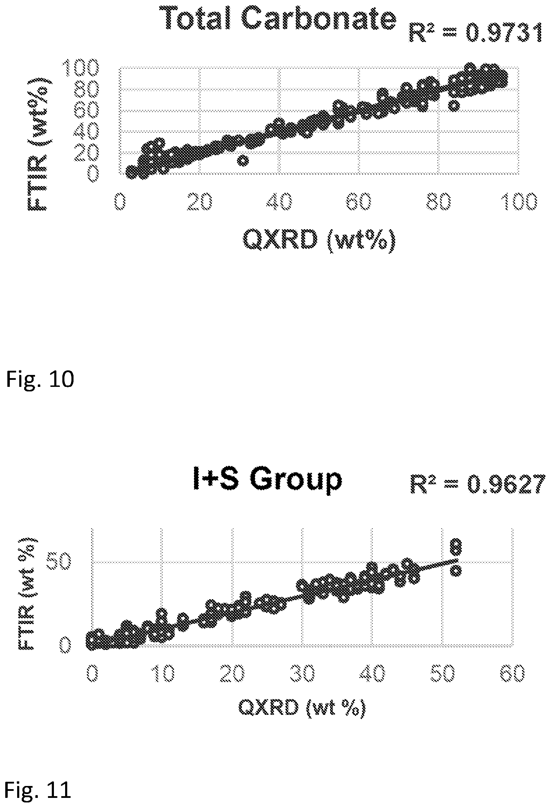

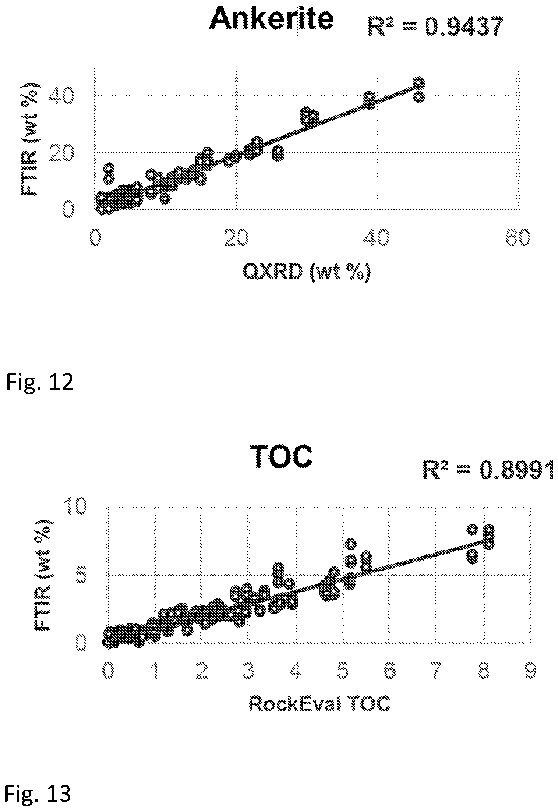

Figures 8 to 15 illustrate how accurately FTIR measurements can be fit to QXRD and Rock-Eval reference measurements when training the calibration model for the new spectroscopic method. In particular, the weight percentage amount of quartz, illite-smectite (I-S) group minerals, feldspar, ankerite, total carbonate and TOC, as well as Rock-Eval parameters S1 (i.e. the height of the first (i.e. S1) peak in a Rock-Eval pyrolysis spectrum, which is a measure of the volume of free hydrocarbons (i.e. gas and oil) present in a sample) and S2 (i.e. the height of the second (i.e. S2) peak in a Rock-Eval pyrolysis spectrum, which is a measure of the volume of hydrocarbons formed during thermal pyrolysis (i.e. thermal cracking of nonvolatile organic matter)), were measured for each sample. As can be seen, there is a strong correlation (as quantified by the coefficient of determination, $R^2$) between the FTIR results and the QXRD or Rock-Eval measurements, although the correlations tend to become less precise as the volume of a rock component falls below 5%.

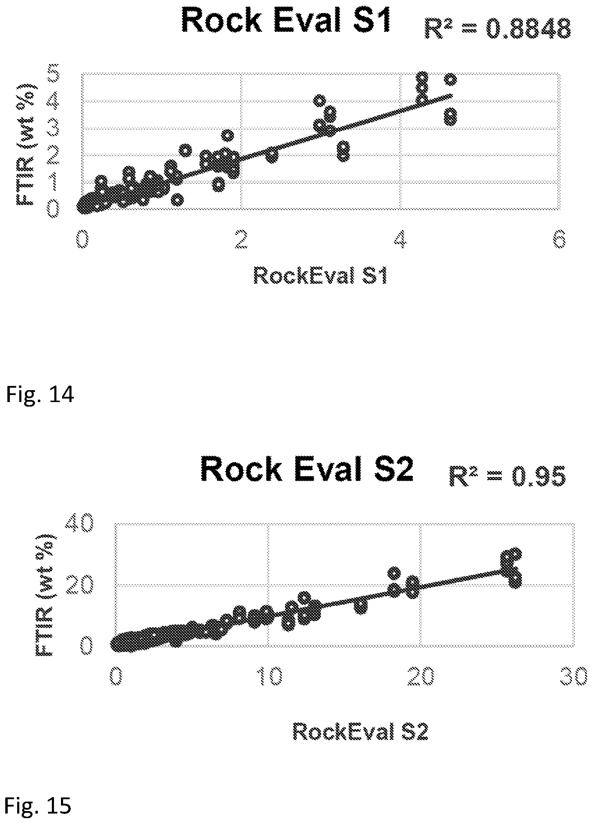

The inventors have found that the initial calibration of the model is important. For example, Figures 15 (a) to (c) show how the coefficient of determination, $R^2$, increases as more reference rock samples are included when compiling the training data set. As mentioned hereinabove, a minimum of around 30 different samples in the training data set is typically required to build to a predictive model.

The inventors have also found that results can be improved for measurements taken in a particular area by including multiple samples taken from the "zone of interest" when compiling the training data set. In particular, it is important that the training data set includes samples having relatively similar mineralogies to (i.e. having the same mineral phases within the same compositional ranges as) the rock found in the area to be analysed. For example, the inventors found errors of up to 20 % in terms of the predicted quartz content for a sample taken from a hydrocarbon well in one sedimentary basin when using an FTIR model calibrated based on a training set compiled using samples taken from a different sedimentary basin. It would therefore be preferable to compile a specific FTIR calibration model for each basin or region of a basin which rock samples are to be analysed.

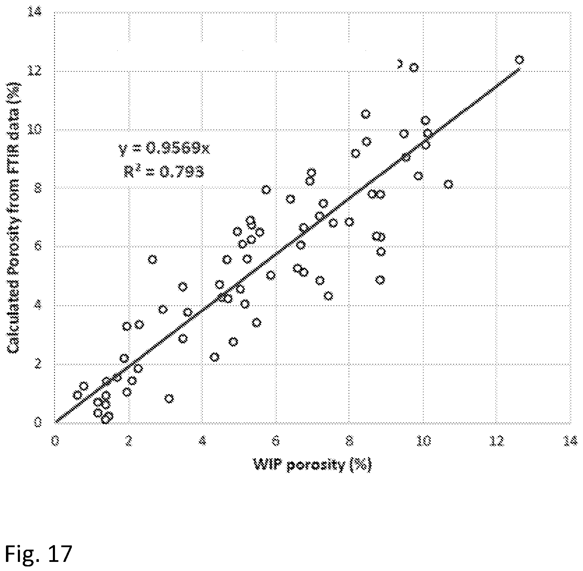

As mentioned hereinabove under *Porosity,* rock matrix density can also be obtained using the calibrated spectroscopic methodology. In particular, spectroscopy in combination with the calibration model can be used to determine the volume fractions of the various mineral and organic phases present in a sample, and then Equation (39) can be applied, taking into account the known densities of each of the phases, to determine $\rho_M$ directly. Figure 17 shows a good agreement the rock porosity determined using calibrated FTIR data for $\rho_M$ and that measured directly using water immersion porosimetry (WIP).

*Results*

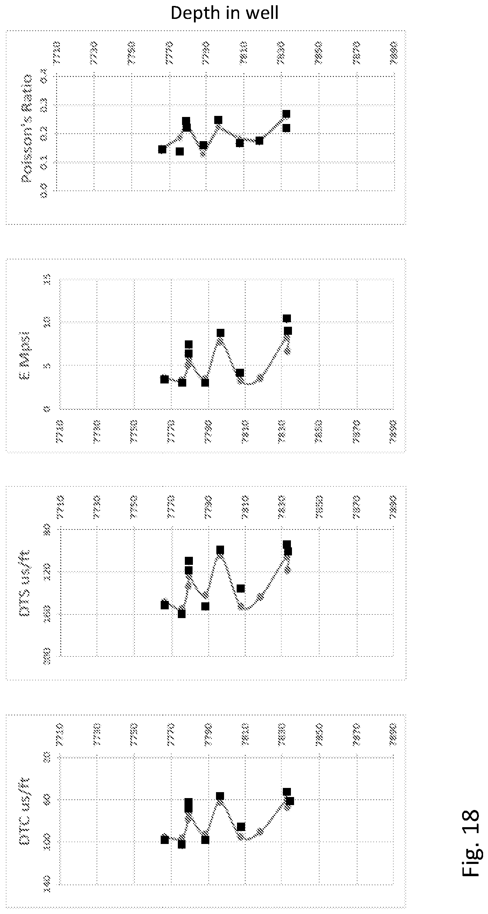

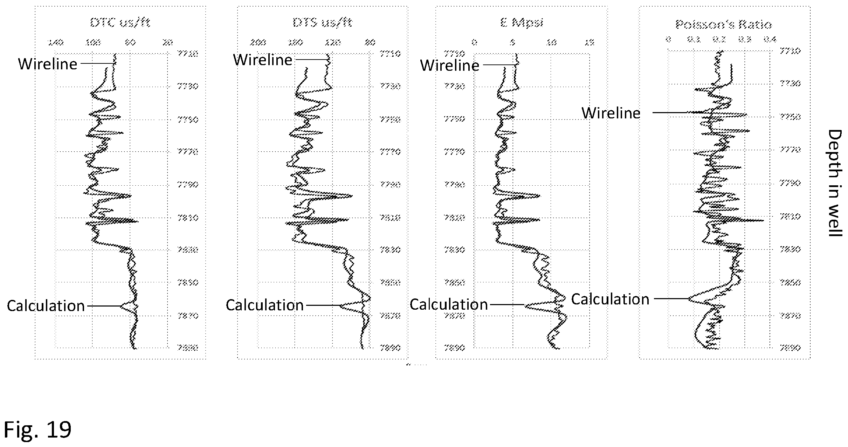

Figure 18 compares values of DTC, DTS, Young's modulus and Poisson's ratio measured directly (i.e. by direct ultrasonic and mechanical property testing) and corresponding values calculated by applying Equations (22) to (31) for a series of core samples obtained at different depths in a hydrocarbon well in the Marcellus formation. The calculated values were based on rock compositions determined precisely using QXRD, porosities determined based on WIP, and reference values for the dynamic and static mechanical properties of the individual constituent phases identified in the rock. As can be seen in Figure 18, there is good agreement between the calculated values (shown as filled circles connected by a solid line) and those measured directly (shown as filled squares). Figure 19 also compares the results of DTC, DTS, Young's modulus and Poisson's ratio determined by calculation using QXRD and WIP data, and those determined by wireline measurements, such as sonic logs measuring the velocity of sound in the rocks surrounding the borehole, along a longer section of the well.

Again, the agreement is good. These results indicate that, with accurate compositional input, it is possible to calculate the dynamic properties (i.e., DTC and DTS) for a given sample, and therefore it is also possible to calculate static mechanical properties (such as Young's modulus and Poisson's ratio).

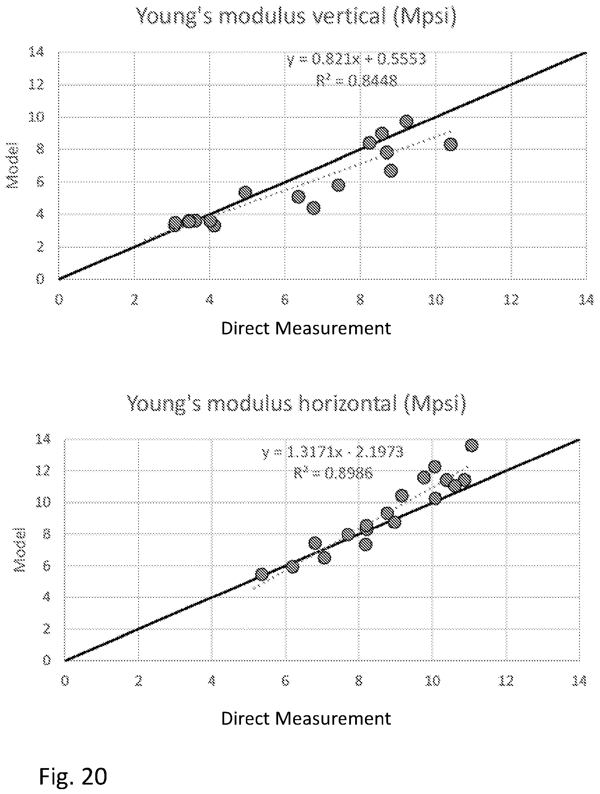

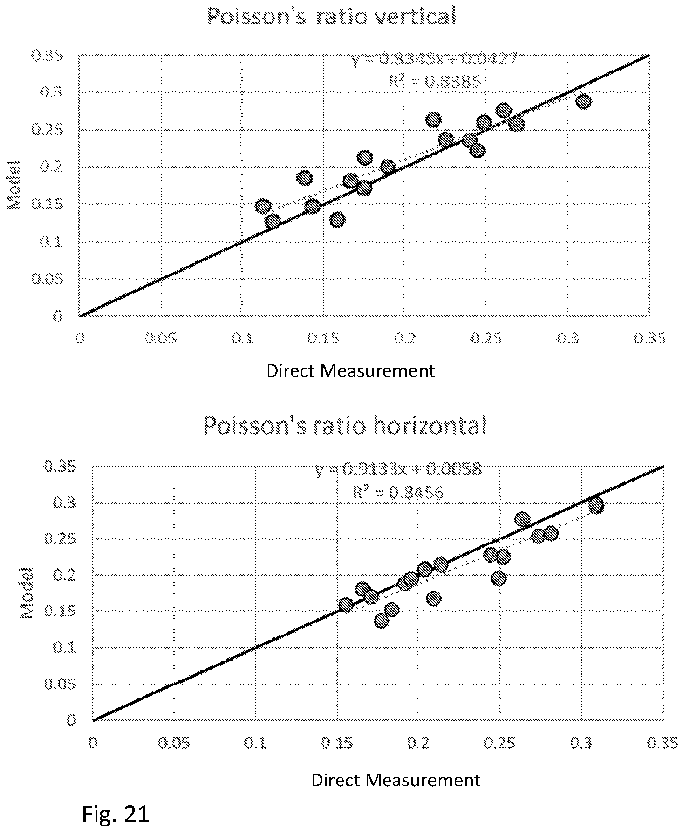

Figures 20 and 21 illustrate the level of accuracy achieved for static property calculations using such methods. In particular, Figure 20 compares calculated and directly measured values of Young's modulus in the vertical and horizontal directions for shale rock samples. Similarly, Figure 21 compares calculated and directly measured values of Poisson's ratio in the vertical and horizontal directions.

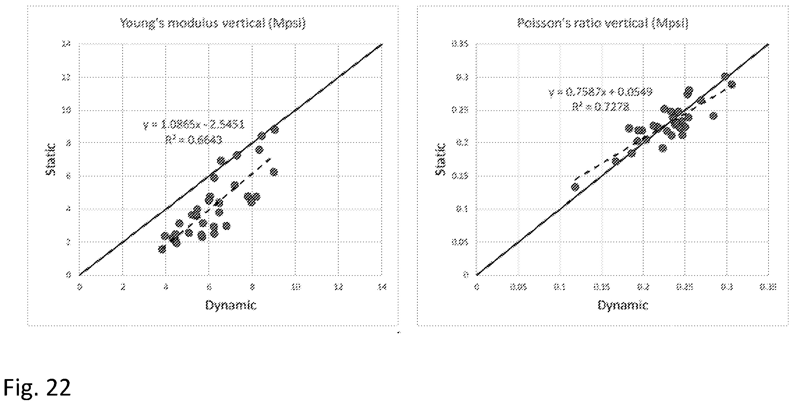

Figure 22 shows the results of comparing dynamic and static Young's modulus and Poisson's ratio for core samples taken from the Delaware Basin. The static values were determined using indentation-based mechanical property testing methods. The dynamic values were determined using ultrasonic measurement methods. The linear relationship between the measured values further illustrates that static elastic moduli can be estimated accurately based on acoustic measurements, and therefore also based on modelled dynamic values.

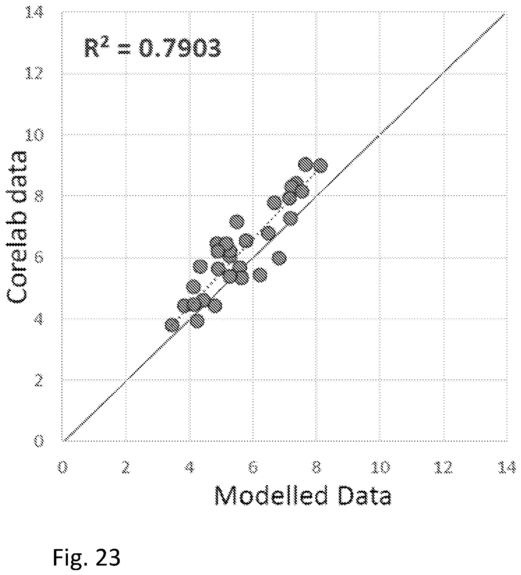

Figure 23 compares values of Young's modulus as measured directly and as calculated based on calibrated FTIR measurements, as described hereinabove, for 30 different samples taken from the Delaware Basin. As can be seen, there is good agreement between the FTIR-based calculations and experiment.

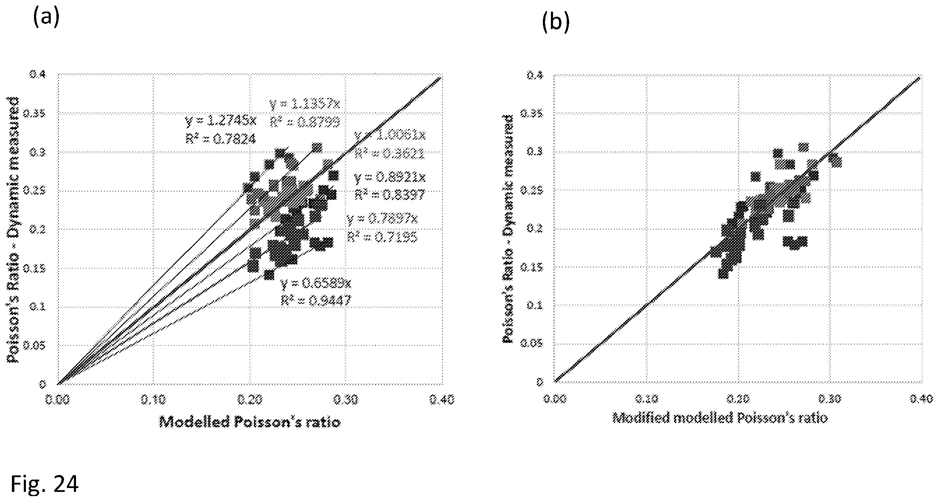

Figure 24 (a) compares values of Poisson's ratio as measured directly and as calculated based on calibrated FTIR measurements, as described hereinabove, for a plurality of core samples taken from the Delaware Basin. As can be seen, there does not appear to be a strong correlation between the modelled values and the experimental data. However, the data can be portioned into groups around straight lines of different gradients, suggesting a systematic error in the modelling approach. The inventors found that in order to collapse the data around the 1:1 correlation lines, it was necessary to remove anisotropy from the calculation by averaging anisotropic input values for the illitic clays used in the model and modifying dynamic moduli values for I-S clays, resulting in the graph shown in Figure 24 (b). Three outlying data points which were not collapsed onto the 1:1 correlation line were found to relate to limestone samples which contained little clay.

The skilled person will appreciate that the methods and calculations described hereinabove are suited to automation and implementation in computer software (for example, computer software 102 stored on a computer-readable medium 101, for execution by a computer processor 100, as shown in Figure 26).

*Anisotropy*

So far it has been assumed that isotropic equations can be used to model the mechanical behaviour of rock samples in each of the horizontal and vertical directions. However, as discussed hereinabove, rocks are generally anisotropic and the application of isotropic equations to each direction is a simplification. Anisotropy in sedimentary rocks, in particular, arises primarily due to the presence of clay minerals and authigenic quartz cement. The accuracy of mechanical property calculations, particularly for sedimentary rocks, could therefore be improved further by taking into account a measurement of rock anisotropy. The present inventors have therefore explored two methods for assessing the anisotropy of rock samples: indentation-based methods and magnetic susceptibility-based methods.

Indentation-based methods can be used to measure directly the anisotropy in the mechanical properties of a rock. For example, a rock sample can be subjected to indentation-based testing in order to measure the Young's modulus (or other mechanical property) in the horizontal direction and the vertical direction (i.e. parallel to and perpendicular to the bedding of the rock). If the same values of Young's modulus are obtained in the horizontal and vertical directions, the rock is isotropic in terms of mechanical properties. However, if different values are obtained for each direction, the rock is anisotropic, and the difference between the two measurements can be taken as a measure of the anisotropy. In some examples, the measure of anisotropy is obtained by plotting the elastic modulus measured in the horizontal direction against the elastic modulus measured in the vertical direction for the same sample, and then measuring a distance of the sample data point from the 1:1 line which represents the line of isotropy, the distance of the sample data point from this 1:1 line being the measure of anisotropy. Geomechanical anisotropy measurements based on indentation measurements in two orthogonal directions quantify the impact of excess silica in a sample in the form of secondary quartz cement.

Nanoindentation mechanical testing devices (such as those provided with a piezoelectric controller for controlling load application and a high precision capacitive ring for measuring indentation depth, for example conforming to the ASTM E2546 and ISO 14577 standards) are particularly suitable for this type of measurement. Nanoindentation can yield hardness and modulus measurements from the generated load/displacement data. However, elastic moduli can also be measured using microindentation methods, in which a sensor is forced into the rock until it fails.

As an alternative to indentation-based techniques, anisotropy can be determined from anhysteric magnetic susceptibility (AMS) measurements. In AMS methods, the magnetic susceptibility of a rock sample is measured in three directions (referred to as $K_{max}$, $K_{int}$ and $K_{min}$) and the degree of magnetic anisotropy in a sedimentary rock (whose magnetic susceptibility is controlled by the rock's mineralogy) is defined as the ratio between $K_{max}$ and $K_{min}$. AMS methods provide a measure of the sedimentological anisotropy caused by the alignment of clay minerals in a sample.

Both indentation-based techniques and AMS-based techniques may be used to measure the anisotropy of core samples. However, the direct application of these techniques to cuttings samples is more challenging. For example, AMS measurements must be carried out on oriented rock samples, whereas the rock chips present in cuttings samples are not oriented. The present inventors, however, have found that measurements of anisotropy may be obtained from cuttings samples by determining the composition of a rock sample using the calibrated spectroscopic method described hereinabove and then calculating the anisotropy of the sample based on the determined composition and known values of indentation-based or AMS-based anisotropy parameters for each of the constituent phases present.

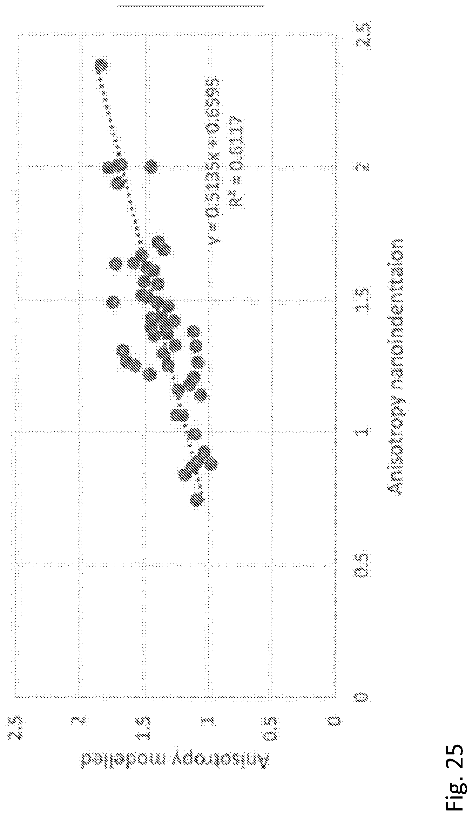

For example, Figure 25 compares the results of direct nanoindentation-based anisotropy measurements and values of the anisotropy obtained from suitably calibrated FTIR measurements for the same samples. There is good agreement between the results.

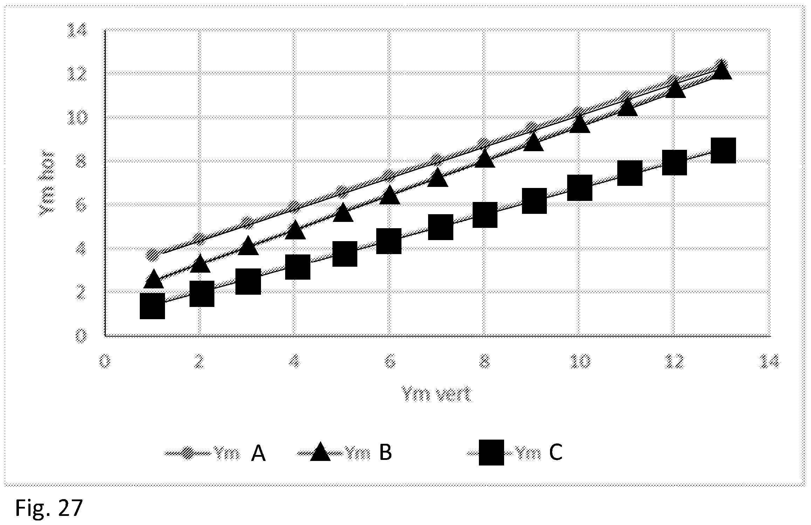

Figure 27 is a plot comparing the static Young's modulus in the horizontal and vertical directions obtained by standard rock mechanic measurements from multiple samples taken from an unconventional basin from multiple stratigraphic intervals and locations (line A labelled with circles). This plot indicates mechanical anisotropy in the measurements. In particular, the gradient of the line is a measure of the degree of mechanical anisotropy - when the gradient is 1, the samples are anisotropic. Line B (labelled with triangles) and line C (labelled with squares) show corresponding horizontal and vertical Young's modulus values as calculated from indentation data obtained from two cores from the same unconventional basin but at different stratigraphic intervals and different locations (north and south of the basin, respectively). The cores used to produce lines B and C were obtained from approximately the same stratigraphic interval. These plots also show mechanical anisotropy. In particular, the three lines have the following equations:

Line A:

$$E_{\mathrm{horizontal}} = 0.723 * E_{\mathrm{vertical}} + 3.0$$

Line B:

$$E_{\mathrm{horizontal}} = 0.7958 * E_{\mathrm{vertical}} + 1.69$$

Line C:

$$E_{\mathrm{horizontal}} = 0.5941 * E_{\mathrm{vertical}} + 0.87$$

Figure 27 indicates that indentation (or a modified version thereof) can provide a means of estimating, and therefore correcting for, anisotropy when calculating Young's modulus and Poisson's ratio from cuttings mineralogy, organic matter and total porosity measurements. In particular, when modelling for anisotropy it can be assumed that anisotropy is reflected by platy minerals (i.e. clays) and organic matter aligning at horizontally (i.e. bedding) or at 90° to the vertical. By measuring the gradient of the indentation plot, the degree of anisotropy can be modelled based on the percentage of a perfectly anisotropic sample, i.e., samples taken from wells near to line C would be more anisotropic (59.41%) than samples nearer to line B (79.58%) because the gradient of the lines has moved further from 1.

It will be understood that the invention is not limited to the embodiments described above and various modifications and improvements can be made without departing from the concepts described herein. Except where mutually exclusive, any of the features may be employed separately or in combination with any other features and the disclosure extends to and includes all combinations and sub-combinations of one or more features described herein.

For the avoidance of doubt, the application extends to the subject-matter set out in the following numbered paragraphs ("Para" or "Paras"):

1. A method comprising determining a mechanical property of a rock sample taking into account (a) a respective amount of each of two or more constituent phases in the rock sample and (b) a corresponding mechanical property parameter associated with each of the two or more constituent phases.

2. The method according to Para 1, wherein determining the mechanical property of the rock sample comprises evaluating a weighted sum of the mechanical property parameters associated with each of the two or more constituent phases, the mechanical property parameter associated with each constituent phase being weighted in the weighted sum by the amount of said constituent phase in the rock sample.

3. The method according to Para 1 or Para 2 comprising:

determining the respective amount of each of the two or more constituent phases in the rock sample; and calculating the mechanical property of the rock sample taking into account (a) the respective determined amount of each of the two or more constituent phases in the rock sample and (b) the corresponding mechanical property parameter associated with each of the two or more constituent phases.

4. The method according to Para 3, wherein determining the respective amount of each of the two or more constituent phases in the rock sample comprises determining the amount of at least one constituent phase in the rock sample by a spectroscopic method, for example by an infra-red spectroscopic method such as Fourier Transform Infra-red (FTIR) spectroscopy.

5. The method according to Para 4, wherein determining the amount of the at least one constituent phase in the rock sample by the spectroscopic method comprises:

obtaining a spectroscopic measurement from the rock sample; and
determining the amount of the at least one constituent phase in the rock sample based on the spectroscopic measurement and a spectroscopic calibration model which defines a relationship between spectroscopic measurements and constituent phase amounts for rock samples.

6. The method according to any of Paras 3 to 5, wherein the at least one constituent phase is a solid constituent phase such as a mineralogical phase or an organic phase.

7. The method according to any of Paras 3 to 6, wherein determining the respective amount of each of the two or more constituent phases in the rock sample comprises:

determining a parameter indicative of the porosity of the rock sample, for example by a water immersion porosimetry (WIP) method; and
determining the amount of at least one liquid phase in the rock sample based on the parameter indicative of the porosity of the rock sample.

8. The method according to Para 7, wherein the at least one liquid phase is a hydrocarbon phase or an aqueous phase such as water.

9. The method according to any preceding Para , wherein the rock sample is a cuttings sample.

10. The method according to any preceding Para, wherein the amount of a constituent phase in the rock sample is a parameter indicative of a volume of the said constituent phase in the rock sample, such as a volume or a volume fraction of the said constituent phase in the rock sample.

11. The method according to any preceding Para , wherein the mechanical property of the rock sample is an elastic modulus such as a Young's modulus, a shear modulus or a bulk modulus, or a dimensionless mechanical property ratio such as a Poisson's ratio.

12. The method according to any preceding Para further comprising taking into account an anisotropy factor associated with the rock sample when determining the mechanical property.

13. The method according to Para 12 further comprising determining the anisotropy factor associated with the rock sample.

14. The method according to Para 13, wherein the anisotropy factor is a mechanical anisotropy factor.

15. The method according to Para 13, wherein the anisotropy factor is a magnetic anisotropy factor.

16. The method according to any preceding Para , wherein the steps of Para 1 or Para 2 are carried out by a computer.

17. A computer program comprising instructions which, when the program is executed by a computer, cause the computer to carry out the steps of the method of Para 1 or Para 2.

18. A computer-readable medium storing the computer program according to Para 17.

19. A method comprising determining a value of an anisotropy parameter indicative of anisotropy in a rock sample based on a spectroscopic measurement obtained from the rock sample.

20. The method according to Para 19, wherein the spectroscopic measurement is obtained by infra-red spectroscopy, for example Fourier Transform Infra-red (FTIR) spectroscopy.

21. The method according to Para 19 or Para 20, wherein determining (measuring) the value of the anisotropy parameter comprises:

obtaining a spectroscopic measurement from the rock sample; and
determining the value of the anisotropy parameter based on the spectroscopic measurement and a spectroscopic calibration model which defines a relationship between spectroscopic measurements and the anisotropy parameter for rock samples.

22. The method according to Para 21, wherein the anisotropy parameter is a mechanical anisotropy parameter indicative of mechanical anisotropy in the rock sample and the spectroscopic calibration model defines a relationship between spectroscopic measurements and mechanical anisotropy parameter measurements for rock samples.

23. The method according to Para 21, wherein the anisotropy parameter is a magnetic anisotropy parameter indicative of magnetic anisotropy in the rock sample and the spectroscopic calibration model defines a relationship between spectroscopic measurements and magnetic anisotropy parameter measurements for rock samples.

24. The method according to any of Paras 19 to 23, wherein the rock sample is a cuttings sample.

25. A method comprising:

providing spectroscopic measurement data obtained from a plurality of reference rock samples;
providing corresponding anisotropic parameter measurement data obtained from the same plurality of reference rock samples; and
fitting a spectroscopic calibration model to the spectroscopic measurement data and the corresponding anisotropic parameter measurement data, wherein the spectroscopic calibration model defines a relationship between the spectroscopic measurement data and the corresponding anisotropic parameter measurement data for the plurality of reference rock samples.

26. The method according to Para 25, wherein the spectroscopic measurement data is infra-red spectroscopic measurement data, for example Fourier Transform Infra-red (FTIR) spectroscopic measurement data.

27. The method according to Para 25 or Para 26, wherein the anisotropic parameter measurement data is mechanical anisotropic parameter measurement data, for example mechanical indentation-based anisotropic parameter measurement data.

28. The method according to Para 25 or Para 26, wherein the anisotropic parameter measurement data is magnetic anisotropic parameter measurement data, for example anhysteric magnetic susceptibility-based magnetic anisotropic parameter measurement data.

29. The method according to any of Paras 25 to 28, wherein the method is carried out by a computer.

30. A computer program comprising instructions which, when the program is executed by a computer, cause the computer to carry out the steps of the method of any of Paras 25 to 29.

31. A data set comprising the spectroscopic measurement data and/or the anisotropic parameter measurement data of any of Paras 25 to 30 and/or spectroscopic calibration model parameter data on which the spectroscopic calibration model of any of Paras 25 to 30 is parametrised.

32. A computer-readable medium storing the computer program according to Para 30 and/or the data set according to Para 31.

33. A method of determining a parameter indicative of a porosity of a rock sample by immersion porosimetry, the method comprising:

measuring a mass of the rock sample when the rock sample is saturated by and submerged in a liquid, thereby obtaining the saturated, submerged mass of the rock sample;

measuring a mass of the rock sample in air when the rock sample is saturated by a liquid, thereby obtaining the saturated mass of the rock sample in air;

measuring a mass of the rock sample in air when the rock sample is dry, thereby obtaining the dry mass of the rock sample in air; and

calculating the parameter indicative of the porosity of the rock sample based on the saturated, submerged mass of the rock sample, the saturated mass of the rock sample in air, the dry mass of the rock sample in air and the density of the liquid.

34. A method of determining a parameter indicative of a bulk density of a rock sample by immersion porosimetry, the method comprising:

measuring a mass of the rock sample when the rock sample is saturated by and submerged in a liquid, thereby obtaining the saturated, submerged mass of the rock sample;

measuring a mass of the rock sample in air when the rock sample is saturated by a liquid, thereby obtaining the saturated mass of the rock sample in air;

measuring a mass of the rock sample in air when the rock sample is dry, thereby obtaining the dry mass of the rock sample in air; and

calculating the parameter indicative of the bulk density of the rock sample based on the saturated, submerged mass of the rock sample, the saturated mass of the rock sample in air, the dry mass of the rock sample in air and the density of the liquid.

35. A method of determining a parameter indicative of a matrix density of a rock sample by immersion porosimetry, the method comprising:

measuring a mass of the rock sample when the rock sample is saturated by and submerged in a liquid, thereby obtaining the saturated, submerged mass of the rock sample;

measuring a mass of the rock sample in air when the rock sample is dry, thereby obtaining the dry mass of the rock sample in air; and

calculating the parameter indicative of the matrix density of the rock sample based on the saturated, submerged mass of the rock sample and the dry mass of the rock sample in air.

36. The method according to any of Paras 33 to 35, wherein the rock sample is a cuttings sample.

37. The method according to any of Paras 33 to 36, wherein the liquid is water.

38. The method according to any of Paras 33 to 37 further comprising, prior to measuring the mass of the rock sample, washing the rock sample.

39. The method according to Para 38, wherein washing the rock sample comprises:
washing the rock sample in water and detergent; and sonicating the rock sample in water.

40. A method comprising determining a matrix density of a rock sample taking into account (a) a respective amount of each of two or more constituent phases in the rock sample and (b) a corresponding density of each of the two or more constituent phases.

41. The method according to Para 40, wherein determining the matrix density of the rock sample comprises evaluating a weighted sum of the densities of each of the two or more constituent phases, the density of each constituent phase being weighted in the weighted sum by the amount of said constituent phase in the rock sample.

42. The method according to Para 40 or Para 41 comprising:

determining the respective amount of each of the two or more constituent phases in the rock sample; and calculating the matrix density of the rock sample taking into account (a) the respective determined amount of each of the two or more constituent phases in the rock sample and (b) the corresponding density of each of the two or more constituent phases.

43. The method according to Para 42 further comprising determining the respective amount of each of the two or more constituent phases in the rock sample by a spectroscopic method, for example by an infra-red spectroscopic method such as Fourier Transform Infra-red (FTIR) spectroscopy.

44. The method according to Para 43, wherein determining the respective amount of each of the two or more constituent phases in the rock sample by a spectroscopic method comprises:

obtaining a spectroscopic measurement from the rock sample; and
determining the amount of said constituent phase in the rock sample based on the spectroscopic measurement and a spectroscopic calibration model which defines a relationship between spectroscopic measurements and constituent phase amounts for rock samples.

45. The method according to any of Paras 40 to 43, wherein the rock sample is a cuttings sample.

46. The method according to any of Paras 40 to 45, wherein the steps of Paras 40 or 41 are carried out by a computer.

47. A computer program comprising instructions which, when the program is executed by a computer, cause the computer to carry out the steps of the method of Para 40 or Para 42.

48. A computer-readable medium storing the computer program according to Para 47.

**Claims**

1. A method comprising determining a mechanical property of a rock sample taking into account (a) a respective amount of each of two or more constituent phases in the rock sample and (b) a corresponding mechanical property parameter associated with each of the two or more constituent phases.

2. The method according to claim 1, wherein determining the mechanical property of the rock sample comprises evaluating a weighted sum of the mechanical property parameters associated with each of the two or more constituent phases, the mechanical property parameter associated with each constituent phase being weighted in the weighted sum by the amount of said constituent phase in the rock sample.

3. The method according to claim 1 or claim 2, wherein the method comprises:

determining the respective amount of each of the two or more constituent phases in the rock sample; and
calculating the mechanical property of the rock sample taking into account (a) the respective determined amount of each of the two or more constituent phases in the rock sample and (b) the corresponding mechanical property parameter associated with each of the two or more constituent phases.

4. The method according to claim 3, wherein determining the respective amount of each of the two or more constituent phases in the rock sample comprises determining the amount of at least one constituent phase in the rock sample by a spectroscopic method, for example by an infra-red spectroscopic method such as Fourier Transform Infra-red (FTIR) spectroscopy.

5. The method according to claim 4, wherein determining the amount of the at least one constituent phase in the rock sample by the spectroscopic method comprises:

obtaining a spectroscopic measurement from the rock sample; and
determining the amount of the at least one constituent phase in the rock sample based on the spectroscopic measurement and a spectroscopic calibration model which defines a relationship between spectroscopic measurements and constituent phase amounts for rock samples.

6. The method according to claim 4 or claim 5, wherein:

(1) the at least one constituent phase is a solid constituent phase such as a mineralogical phase or an organic phase; and/or
(2) determining the respective amount of each of the two or more constituent phases in the rock sample comprises:

determining a parameter indicative of the porosity of the rock sample, for example by a water immersion porosimetry (WIP) method; and

determining the amount of at least one liquid phase in the rock sample based on the parameter indicative of the porosity of the rock sample,

optionally wherein the at least one liquid phase is a hydrocarbon phase or an aqueous phase such as water.

7. The method according to any preceding claim, wherein:

(i) the mechanical property parameters associated with each of the two or more constituent phases are dynamic mechanical property parameters, such as velocities of acoustic waves or acoustic wave travel times in each of the two or more constituent phases;

(ii) the mechanical property of the rock sample is a dynamic mechanical property of the rock sample, such as such a velocity of an acoustic wave or an acoustic wave travel time in the rock sample; and/or

(iii) the method comprises determining a static mechanical property of the rock sample by: determining one or more dynamic mechanical properties of the rock sample; and determining the static mechanical property of the rock sample based on the one or more determined dynamic mechanical properties.

8. The method according to any of claims 1 to 6 or 7 (i) or 7 (iii), wherein the mechanical property of the rock sample is an elastic modulus such as a Young's modulus, a shear modulus or a bulk modulus, or a dimensionless mechanical property ratio such as a Poisson's ratio.

9. The method according to any preceding claim, wherein the method further comprises taking into account an anisotropy factor associated with the rock sample when determining the mechanical property, optionally wherein the method further comprises determining the anisotropy factor associated with the rock sample, further optionally wherein the anisotropy factor is a mechanical anisotropy factor or a magnetic anisotropy factor.

10. The method according to any preceding claim, wherein the rock sample is a cuttings sample.

11. The method according to any preceding claim, wherein the amount of a constituent phase in the rock sample is a parameter indicative of a volume of the said constituent phase in the rock sample, such as a volume or a volume fraction of the said constituent phase in the rock sample.

12. The method according to any preceding claim, wherein the steps of claim 1 or claim 2 are carried out by a computer.

13. A computer program comprising instructions which, when the program is executed by a computer, cause the computer to carry out the steps of the method of claim 1 or claim 2.

14. A computer-readable medium storing the computer program according to claim 13.

Fig. 1

| Property | Calcite | Dolomite | Quartz | Microcline | Plagioclase | Hematite | Siderite | Anhydrite | Pyrite | Halite | Apatite |
|---|---|---|---|---|---|---|---|---|---|---|---|
| Density (kg/m3) | 2712 | 2981 | 2651 | 2537 | 2619 | 5254 | 3926 | 2970 | 5013 | 2164 | 3129 |
| Bulk modulus (Gpa) | 70.2 | 94.90 | 37.8 | 55.4 | 62.87 | 106.5 | 115.01 | 54.9 | 142.7 | 24.9 | 84.02 |
| Shear modulus (Gpa) | 29 | 45.70 | 44.3 | 26.1 | 29.45 | 91 | 46.06 | 29.3 | 125.7 | 14.7 | 57.38 |
| Young's modulus (Gpa) | 76.47 | 118.14 | 95.57 | 72.11 | 76.42 | 238.05 | 126.69 | 74.62 | 291.51 | 36.85 | 140.22 |
| Poisson's ratio | 0.32 | 0.29 | 0.06 | 0.28 | 0.30 | 0.31 | 0.32 | 0.27 | 0.16 | 0.25 | 0.22 |

Fig. 2

| Property | Illite/smectite | Chlorite | Kaolinite | Muscovite |
|---|---|---|---|---|
| DENS kg/m3 | 2857 | 2892 | 2622 | 2844 |
| $c_{11}$ GPa | 153.9 | 183.3 | 169.1 | 180.9 |
| $c_{22}$ GPa | 188.5 | 183.3 | 179.7 | 170 |
| $c_{33}$ GPa | 27.2 | 96.8 | 81.1 | 60.3 |
| $c_{44}$ GPa | 10.4 | 11.5 | 17 | 18.4 |
| $c_{55}$ GPa | 24.8 | 11.5 | 26.6 | 23.8 |
| $c_{66}$ GPa | 55.4 | 57.2 | 57.6 | 70.5 |
| $c_{12}$ GPa | 25.1 | 68.9 | 66.1 | 53.4 |
| $c_{13}$ GPa | 13.2 | 39.68 | 15.4 | 27.2 |

Fig. 3

| Property | Organic matter | Organic matter |
|---|---|---|
| Density (kg/m3) | 1300 | 1300 |
| Bulk modulus (Gpa) | 2.9 | 6 |
| Shear modulus (Gpa) | 2.7 | 3 |
| Young's modulus (Gpa) | 6.18 | 7.71 |
| Poisson's ratio | 0.14 | 0.29 |

Fig. 4

| Property | Water | Hydrocarbon |
|---|---|---|
| Density (kg/m³) | 1000 | 262 |
| Vp (km/s) | 1.52 | 0.60 |
| Vs (km/s) | 0.95 | 0.95 |
| DTC* (us/m) | 656 | 1667 |
| DTS* (us/m) | 1050 | 1050 |

Fig. 5

**Bulk Density Comparison**

$y = 1.0015x$
$R^2 = 0.8907$

Bulk Density (core samples)

Bulk Density (cuttings samples)

Fig. 6

Fig. 7

Fig. 8

Fig. 9

**Total Carbonate** R² = 0.9731

Fig. 10

**I+S Group** R² = 0.9627

Fig. 11

**Ankerite** $R^2 = 0.9437$

Fig. 12

**TOC** $R^2 = 0.8991$

Fig. 13

## Rock Eval S1   R² = 0.8848

Fig. 14

## Rock Eval S2   R² = 0.95

Fig. 15

Fig. 16

(a)

(b)

(c)

Fig. 17

## Depth in well

Fig. 18

Depth in well

Fig. 19

Young's modulus vertical (Mpsi)

$y = 0.821x + 0.5553$

$R^2 = 0.8448$

Direct Measurement

Young's modulus horizontal (Mpsi)

$y = 1.3171x - 2.1973$

$R^2 = 0.8986$

Direct Measurement

Fig. 20

Poisson's ratio vertical

$y = 0.8345x + 0.0427$
$R^2 = 0.8385$

Direct Measurement

Poisson's ratio horizontal

$y = 0.9133x + 0.0058$
$R^2 = 0.8456$

Direct Measurement

Fig. 21

Fig. 22

Fig. 23

(a)

(b)

Fig. 24

Fig. 25

100

102

101

Fig. 26

Fig. 27

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MILITZER, B et al.** First-principles calculation of the elastic moduli of sheet silicates and their application to shale anisotropy. *American Mineralogist*, 2011